(19) Europäisches Patentamt
European Patent Office
Office européen des brevets

(11) EP 1 226 826 B1

(12) **EUROPEAN PATENT SPECIFICATION**

(45) Date of publication and mention of the grant of the patent:
**18.07.2007 Bulletin 2007/29**

(21) Application number: **00953450.4**

(22) Date of filing: **17.08.2000**

(51) Int Cl.:
***A61K 31/737*** *(2006.01)* ***A61K 36/02*** *(2006.01)*
***A61K 35/56*** *(2006.01)* ***A61P 37/02*** *(2006.01)*
***A61P 43/00*** *(2006.01)* ***A61P 37/08*** *(2006.01)*
***C08B 37/00*** *(2006.01)*

(86) International application number:
**PCT/JP2000/005489**

(87) International publication number:
**WO 2001/013925 (01.03.2001 Gazette 2001/09)**

(54) **REMEDIES**

HEILMITTEL

MEDICAMENTS

(84) Designated Contracting States:
**AT BE CH CY DE DK ES FI FR GB GR IE IT LI LU MC NL PT SE**

(30) Priority: **20.08.1999 JP 23426299**
**13.03.2000 JP 2000069223**

(43) Date of publication of application:
**31.07.2002 Bulletin 2002/31**

(73) Proprietor: **TAKARA BIO INC.**
**Otsu-shi,**
**Shiga (JP)**

(72) Inventors:
- **TOMINAGA, Takanari**
  **Otsu-shi,**
  **Shiga 520-0242 (JP)**
- **YAMASHITA, Syusaku**
  **Otsu-shi,**
  **Shiga 520-2135 (JP)**
- **MIZUTANI, Shigetoshi**
  **Shiga 521-1322 (JP)**
- **SAGAWA, Hiroaki**
  **Kusatsu-shi,**
  **Shiga 525-0025 (JP)**
- **KATO, Ikunoshin**
  **Uji-shi,**
  **Kyoto611-0028 (JP)**

(74) Representative: **Vossius & Partner**
**Siebertstrasse 4**
**81675 München (DE)**

(56) References cited:
**EP-A- 0 645 143** **EP-A- 0 916 269**
**EP-A- 1 020 474** **WO-A1-88/05301**
**JP-A- 9 255 577** **JP-A- 10 072 362**

- **UEHARA MEGUMI ET AL: "EFFECTS OF FUCOIDAN EXTRACTED FROM OKINAWAMOZUKU (CLADOSIPHON OKAMURANUS TOKIDA) ON THE SERUM CHOLESTEROL LEVELS IN CHOLESTEROL-FED RATS" CHEMICAL ABSTRACTS + INDEXES, AMERICAN CHEMICAL SOCIETY. COLUMBUS, US, vol. 125, no. 13, 23 September 1996 (1996-09-23), XP002185345 ISSN: 0009-2258**
- **CHEMICAL ABSTRACTS, vol. 124, no. 25, 1996, Columbus, Ohio, US; abstract no. 332302, SUN JUYUN ET AL.: 'An experimental study on immunoregulatory effect of fucoidin' XP002935169**
- **GRANERT CARL ET AL.: 'Effects of polysaccharide fucoidin on cerebrospinal fluid interleukin-1 and tumor necrosis factor alpha in pneumococcal meningitus in the rabbit' INFECT. IMMUN. vol. 67, no. 5, 1999, pages 2071 - 2074, XP002935170**
- **YOKOKAWA K. ET AL.: 'Heparin regulates endothelin production through endothelium-derived nitric oxide in human endothelial cells' JOURNAL OF CLINICAL INVESTIGATION vol. 92, no. 4, 1993, pages 2080 - 2085, XP002935171**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

TECHNICAL FIELD

The present invention relates to applications of physiologically active substances derived from aquatic organisms as medicaments, foods, beverages or feeds.

BACKGROUND ART

As physiologically active substances derived from aquatic organisms, a fucoidan has been known. This fucoidan is a sulfated fucose-containing polysaccharide contained in algae, Echinodermata, or the like. Namely, the fucoidan comprises a sulfated fucose as a constituting saccharide.

As physiological action for the fucoidan, cancer proliferation-suppressing activity, cancer metastasis-suppressing activity, anticoagulation activity, antiviral activity and the like have been known, and the developments of applications as medicaments have been expected of the fucoidan.

DISCLOSURE OF INVENTION

The present invention is based on the findings of new physiological action for the fucoidan, and its object is to provide a medicament, food, beverage or feed, utilizing the regulatory action for cytokine production or the like of the fucoidan. In the present specification, the therapeutic agent or prophylactic agent of the present invention is referred to as a medicament in some cases.

Summarizing the present invention, a first invention of the present invention relates to a therapeutic agent or prophylactic agent for a disease requiring regulation of cytokine production, a disease requiring nitrogen monoxide production, or an allergic disease, characterized in that the therapeutic agent or prophylactic agent comprises as an effective ingredient a fucoidan and/or a degradation product thereof.

A second invention of the present invention relates to a food, beverage or feed for regulation of cytokine production, induction of nitrogen monoxide production, or anti-allergy, wherein the food, beverage or feed comprises a fucoidan and/or a degradation product thereof.

In addition, one embodiment of the present invention relates to use of a fucoidan and/or a degradation product thereof for treating or preventing a disease requiring regulation of cytokine production, a disease requiring nitrogen monoxide production, or an allergic disease; use of a fucoidan and/or a degradation product thereof for production of a therapeutic agent or prophylactic agent for a disease requiring regulation of cytokine production, a disease requiring nitrogen monoxide production, or an allergic disease; or a method of treating or preventing a disease requiring regulation of cytokine production, a disease requiring nitrogen monoxide production, or an allergic disease, wherein a fucoidan and/or a degradation product thereof is used.

A still another embodiment of the present invention relates to an agent for regulation of cytokine production, induction of nitrogen monoxide production, anti-allergy, or suppression of IgE production, comprising as an effective ingredient a fucoidan and/or a degradation product thereof.

A still another embodiment of the present invention relates to use of a fucoidan and/or a degradation product thereof in the manufacture of an agent for regulation of cytokine production, induction of nitrogen monoxide production, anti-allergy agent, or suppression of IgE production.

A still another embodiment of the present invention relates to use of a fucoidan and/or a degradation product thereof in the manufacture of a food, beverage or feed for regulation of cytokine production, induction of nitrogen monoxide production, or anti-allergy.

A still another embodiment of the present invention relates to a method for regulating cytokine production, inducing nitrogen monoxide production, suppressing allergy, or suppressing IgE production, wherein a fucoidan and/or a degradation product thereof is used as an effective ingredient.

A still another embodiment of the present invention relates to use of a fucoidan and/or a degradation product thereof for regulating cytokine production, inducing nitrogen monoxide production, suppressing allergy or suppressing IgE production.

In a further preferred embodiment of the present invention, a fucoidan and/or a degradation product thereof has a remarkable effect in the regulation of cytokine production in sensitization with an antigen, for instance, in presentation of an antigen from antigen-presenting cells to T cells. Also, a fucoidan and/or a degradation product thereof is remarkably effective in the treatment of an allergic disease after sensitization with an antigen, especially effective in suppression of antigen-specific IgE production after sensitization with an antigen. In the present invention, the regulation of cytokine production includes regulation of the produced amount of cytokine by the enhancement, acceleration or the like of cytokine production.

Therefore, one embodiment of the present invention relates to a therapeutic agent or prophylactic agent for a disease requiring regulation of cytokine production during sensitization with an antigen, or an allergic disease after sensitization with an antigen, characterized in that the therapeutic agent or prophylactic agent comprises as an effective ingredient a fucoidan and/or a degradation product thereof.

In addition, one embodiment of the present invention relates to a food, beverage or feed for regulation of cytokine production during sensitization with an antigen, or anti-allergy after sensitization with an antigen, wherein the food, beverage or food comprises a fucoidan and/or a degradation product thereof.

Another embodiment of the present invention relates to use of a fucoidan and/or a degradation product thereof for treating or preventing a disease requiring regulation of cytokine production during sensitization with an antigen, or an allergic disease after sensitization with an antigen; use of a fucoidan and/or a degradation product thereof in the manufacture of a therapeutic agent or prophylactic agent for a disease requiring regulation of cytokine production during sensitization with an antigen, or an allergic disease after sensitization with an antigen; or a method of treating or preventing a disease requiring regulation of cytokine production during sensitization with an antigen, or an allergic disease after sensitization with an antigen, wherein a fucoidan and/or a degradation product thereof is used.

A still another embodiment of the present invention relates to an agent for regulation of cytokine production during sensitization with an antigen, anti-allergy after sensitization with an antigen, or suppression of IgE production after sensitization with an antigen, comprising as an effective ingredient a fucoidan and/or a degradation product thereof.

A still another embodiment of the present invention relates to use of a fucoidan and/or a degradation product thereof in the manufacture of an agent for regulation of cytokine production during sensitization with an antigen, anti-allergy after sensitization with an antigen, or suppression of IgE production after sensitization with an antigen.

A still another embodiment of the present invention relates to use of a fucoidan and/or a degradation product thereof in the manufacture of a food, beverage or feed for regulation of cytokine production during sensitization with an antigen, or anti-allergy after sensitization with an antigen.

A still another embodiment of the present invention relates to regulating cytokine production during sensitization with an antigen, suppressing allergy after sensitization with an antigen, or suppressing IgE production after sensitization with an antigen, wherein a fucoidan and/or a degradation product thereof is used as an effective ingredient.

A still another embodiment of the present invention relates to use of a fucoidan and/or a degradation product thereof for regulation of cytokine production during sensitization with an antigen, suppression of allergy after sensitization with an antigen, or suppression of IgE production after sensitization with an antigen.

The fucoidan used in the present invention is not particularly limited. There are preferably exemplified a fucoidan derived from an algae, or Echinodermata.

Also, as the degradation product of the fucoidan, there can be used, for instance, an acid degradation product of a fucoidan, an enzyme degradation product of a fucoidan, and the like.

The fucoidan and a degradation product of the fucoidan, for instance, the acid degradation product of the fucoidan or the enzyme degradation product of the fucoidan, which are used in the present invention, are not particularly limited, as long as they exhibit action for regulation of cytokine production, induction of nitrogen monoxide production, or anti-allergy such as suppression of IgE production. The fucoidan or a degradation product thereof can be appropriately prepared on the basis of its action and the like.

In addition, the term "cytokine" as referred to in the present specification is the cytokine on which the fucoidan or a degradation product thereof can exhibit an action for regulation of production. The cytokine includes, for instance, an interleukin (for instance, interleukin-12), and an interferon (for instance, interferon-γ).

Further, the anti-allergy action refers to action for suppression of allergy which can be exhibited by the fucoidan or a degradation product thereof, which is exemplified by action for suppression of IgE production.

## BRIEF DESCRIPTION OF THE DRAWINGS

Figure 1 is a graph showing an elution pattern of the fucoidan derived from *Kjellmaniella crassifolia* on DEAE-Cellulofine A-800 column.

Figure 2 is a graph showing the $NO_2^-$ concentration in the medium when cells are cultured with adding 7-12SFd-F.

Figure 3 is a graph showing the $NO_2^-$ concentration in the medium when cells are cultured with adding Fraction I.

Figure 4 is a graph showing the $NO_2^-$ concentration in the medium when cells are cultured with adding Fraction II.

Figure 5 is a graph showing the $NO_2^-$ concentration in the medium when cells are cultured with adding Fraction III.

Figure 6 is a graph showing the $NO_2^-$ concentration in the medium when cells are cultured with adding LPS, the positive control.

Figure 7 is a graph showing the action for induction of IFN-γ production by the fucoidan and a degradation product thereof.

Figure 8 is a graph showing the action for induction of IL-12 production by the fucoidan and a degradation product thereof.

Figure 9 is a graph showing the action for cytotoxic immunopotentiation of murine spleen lymphocytes by the fucoidan derived from *Kjellmaniella crassifolia*.

Figure 10 is a graph showing the action for induction of IFN-γ production by G-fucoidan.

Figure 11 is a graph showing the action for induction of IL-12 production by G-fucoidan.

Figure 12 is a graph showing the action of various antibodies against the action for induction of IFN-γ production or IL-12 production by the fucoidan.

Figure 13 is a graph showing the action for induction of IFN-γ production by each fucoidan.

## BEST MODE FOR CARRYING OUT THE INVENTION

The fucoidan used in the present invention refers to a polysaccharide, wherein a sulfated fucose is contained as a constituting saccharide. The fucoidan is not particularly limited, as long as the fucoidan has action for regulation of cytokine production, for instance, action for regulation of interferon-γ production or action for regulation of interleukin-12 during antigen presentation reaction between antigen presenting cells (APCs) and T cells; action for induction of nitrogen monoxide production; or anti-allergic action, for instance, an action for suppression of IgE production. For instance, algae, especially marine algae such as Laminariales, Chordariales and Fucales, including *Kjellmaniella crassifolia, Kjellmaniella gyrata, Fucus, Cladosiphon okamuranus, Undaria, Ecklonia kurome, Eisenia bicyclis, Ecklonia cava,* Giant kelp, *Lessonia nigrescence* and *Ascophyllum nodosum* richly contain fucoidans suitable for the use in the present invention. This is why they are preferable as the raw material. In addition, there may be used fucoidans derived from Echinodermata, for instance, sea cucumber, Echnoidea, Asterozoa, and the like.

These fucoidans may be prepared each by a known method. There can be used in the present invention a purified product of a fucoidan, a fucoidan-containing substance, and the like.

For instance, a fucoidan is prepared from *Kjellmaniella crassifolia*, and the resulting fucoidan can be further separated into glucuronic acid-containing fucoidan (referred to as "U-fucoidan") and glucuronic acid non-containing fucoidan (referred to as "F-fucoidan"). Those fucoidans can be used as an effective ingredient for the therapeutic agent, the prophylactic agent or the like of the present invention. Also, sulfated fucogalactan (referred to as "G-fucoidan") can be prepared from *Kjellmaniella crassifolia* and used.

After the preparation of the fucoidans from *Kjellmaniella crassifolia*, U-fucoidan and F-fucoidan are separated by using an anionic exchange resin, a surfactant or the like. The existing ratio, as expressed by a weight ratio, of U-fucoidan to F-fucoidan derived from *Kjellmaniella crassifolia* is about 1:2. U-fucoidan contains fucose, mannose, galactose, glucuronic acid and the like, and its sulfate content is about 20% by weight. F-fucoidan contains fucose and galactose, and its sulfate content is about 50% by weight. The molecular weight for both substances is distributed, centering about 200000 (Summary of 18th Sugar Symposium, p. 159, 1996).

U-fucoidan and F-fucoidan can be separated, for instance, by applying a fucoidan solution prepared from *Kjellmaniella crassifolia* onto DEAE-Cellulofine A-800 column, and carrying out elution by the concentration gradient technique using NaCl-containing buffer. One of the examples is shown in Figure 1. Concretely, Figure 1 is a diagram showing the separation of U-fucoidan and F-fucoidan, wherein the former peak in the figure is U-fucoidan, and the latter peak is F-fucoidan.

In addition, the previously mentioned G-fucoidan can be obtained by degrading the fucoidan derived from *Kjellmaniella crassifolia* with F-fucoidan-specific degradation enzyme produced by *Alteromonas sp.* SN-1009 (FERM BP-5747), and U-fucoidan-specific degradation enzyme produced by *Flavobacterium sp.* SA-0082 (FERM BP-5402), and removing the degradation product.

Also, for instance, each of the fucoidan derived from *Fucus,* the fucoidan derived from *Cladosiphon okamuranus,* the fucoidan derived from *Undaria,* and the fucoidan derived from sporophyll of *Undaria pinnatifida* (Wakame Mekabu) can be prepared by a known method and used in the present invention.

The fucoidan derived from Echinodermata suitably used in the present invention includes, for instance, fucoidans contained in sea cucumbers described in Japanese Patent Laid-Open No. Hei 4-91027. The fucoidan can be prepared from sea cucumbers in accordance with the method described in the publication.

In addition, the degradation product of the fucoidan having action for regulation of cytokine production, induction of nitrogen monoxide production, and anti-allergy used in the present invention can be prepared by a known method such as an enzymological method, a chemical method, or a physical method, wherein a desired degradation product having the desired action for regulation of cytokine production, induction of nitrogen monoxide production, or anti-allergy is selected. The resulting degradation product can be used.

The method for preparing the degradation product of the fucoidan includes, for instance, an acid degradation method. By subjecting the fucoidan to an acid degradation, there can be prepared a degradation product having action for regulation of cytokine production, immunopotentiation, induction of nitrogen monoxide production, or anti-allergy.

The conditions for the acid degradation in the above-mentioned acid degradation method are not particularly limited, as long as the conditions enable to generate the degradation product having action for regulation of cytokine production, induction of nitrogen monoxide production, or anti-allergy (hereinafter referred to as "degradation product of the present invention").

For instance, the fucoidan is dissolved or suspended in an acid and subjected to the reaction, thereby generating a degradation product of the present invention. Also, the reaction mixture may be heated during the reaction, thereby shortening the time period required for the generation of the degradation product of the present invention.

The kinds of the acids for dissolving or suspending the fucoidan are not particularly limited. There can be used an inorganic acid such as hydrochloric acid, sulfuric acid or nitric acid; an organic acid such as citric acid, formic acid, acetic acid, lactic acid or ascorbic acid; and a solid acid such as cationic exchange resin, cationic exchange fiber or cationic exchange membrane.

The concentration of the acid is not particularly limited, and the acid can be used at a concentration of preferably from 0.0001 to 5 N or so, more preferably from 0.01 to 1 N or so. In addition, the reaction temperature is not particularly limited, and the reaction temperature may be set at preferably from 0° to 200°C, more preferably from 20° to 130°C.

In addition, the reaction time is not particularly limited, and the reaction time may be set at preferably from several seconds to several days. The kinds and the concentration of the acids, the reaction temperature, and the reaction time may be properly selected depending upon the generated amount of the degradation product of the present invention and the degree of polymerization of the degradation product. For instance, during the manufacture of the degradation product of the present invention, it is preferable that an organic acid such as citric acid, lactic acid or malic acid is used, and that the concentration of the acid is properly selected from the range of several dozens mM to several M, and that the heating temperature from the range of 50° to 110°C, more preferably 70° to 95°C, and that the heating time from the range of several minutes to 24 hours, whereby the degradation product of the present invention can be prepared. The acid degradation product of the fucoidan is exemplified by the acid degradation product of the fucoidan derived from *Kjellmaniella crassifolia,* and this degradation product can be used as dietary fiber having new physiological functions of strong action for regulation of cytokine production, especially regulation of interferon-$\gamma$ production during antigen presentation reaction between APCs and T cells; induction of nitrogen monoxide production; and anti-allergy.

The degradation product of the present invention can be fractionated on the basis of its action for regulation of cytokine production, induction of nitrogen monoxide production, or anti-allergy. For instance, the acid degradation product can be further fractionated based on a molecular weight by means of a gel filtration method, a fractionation method using a molecular weight fractionation membrane, or the like.

As an example of gel filtration method, Cellulofine GCL-300 can be used to prepare any molecular weight fractions, for instance, one having a molecular weight exceeding 25000, one having a molecular weight of 25000 to exceeding 10000, one having a molecular weight of 10000 to exceeding 5000, one having a molecular weight of 5000 or less. Cellulofine GCL-25 can be used to prepare any molecular weight fractions from the fraction having a molecular weight of 5000 or less, for instance, one having a molecular weight of 5000 to exceeding 3000, one having a molecular weight of 3000 to exceeding 2000, one having a molecular weight of 2000 to exceeding 1000, one having a molecular weight of 1000 to exceeding 500, one having a molecular weight of 500 or less.

In addition, the molecular weight fractionation can be industrially carried out by using an ultrafiltration membrane. For instance, a fraction having a molecular weight of 30000 or less can be prepared by using FE10-FUSO382 manufactured by DAICEL CHEMICAL INDUSTRIES, LTD., or a fraction having a molecular weight of 6000 or less can be prepared by using FE-FUS-T653 manufactured by the same. Further, a fraction having a molecular weight of 500 or less can be obtained by using a nanofilter membrane. Any molecular weight fractions can be prepared by combining these gel filtration methods and molecular weight fractionation methods.

The degradation product of the fucoidan having action for regulation of cytokine production, induction of nitrogen monoxide, or anti-allergy which can be used in the present invention is exemplified by the compounds represented by the formulas (I) to (IV), and these compounds can be prepared in accordance with the methods disclosed in WO 97/26896, and the specifications for International Application Nos. PCT/JP99/00606 and PCT/JP00/00965. In addition, the degradation product of fucoidan of the present invention encompasses degradation products of fucoidan described in WO 97/26896, and the specifications for International Application Nos. PCT/JP99/00606 and PCT/JP00/00965. A fucoidan and an oligosaccharide having a repeating structure of the compound represented by the formula (I) can be also favorably used in the present invention.

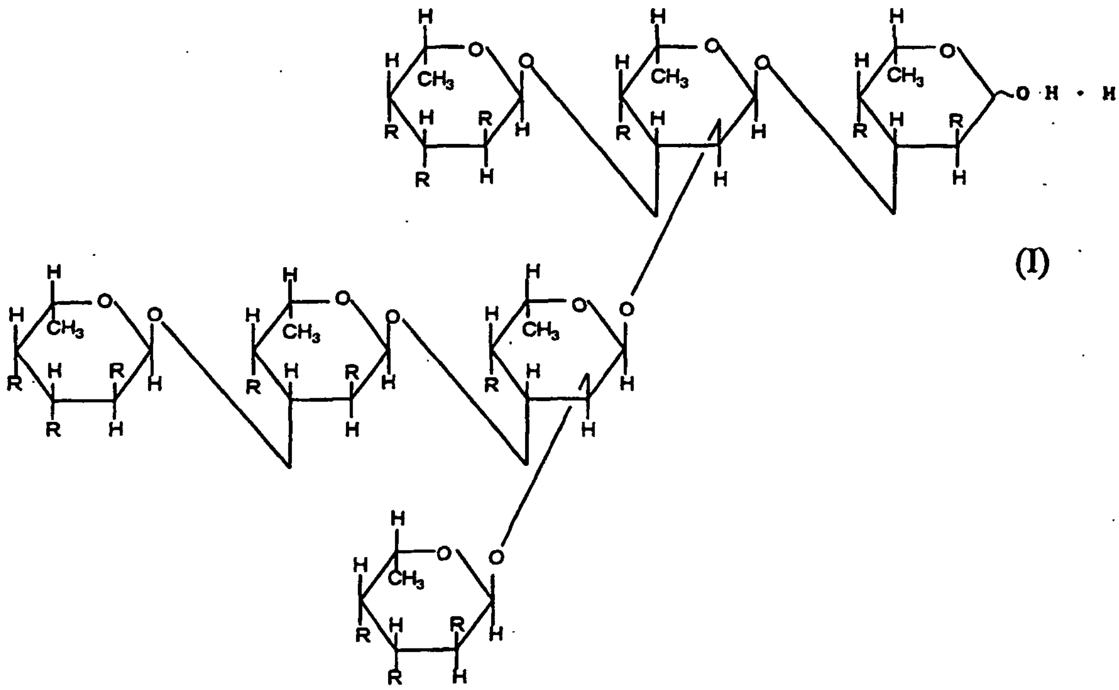

(I)

wherein R is OH or $OSO_3H$;

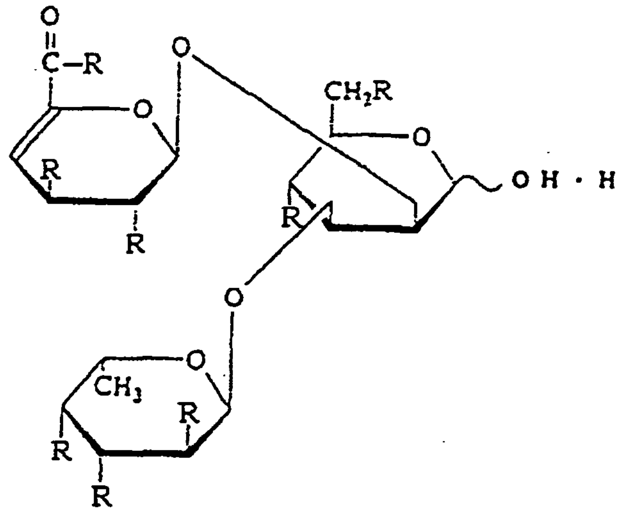

(II)

wherein R is OH or $OSO_3H$;

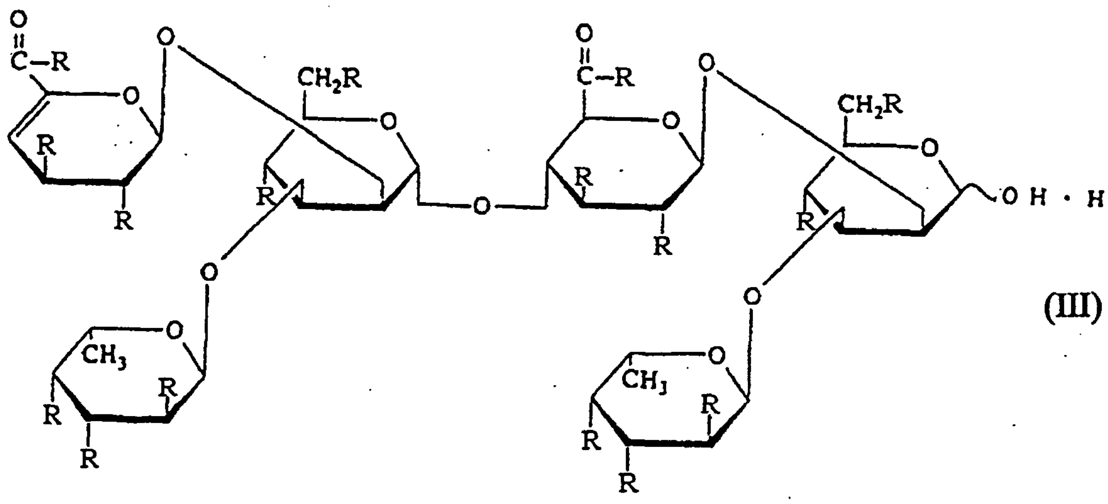

(III)

wherein R is OH or $OSO_3H$; and

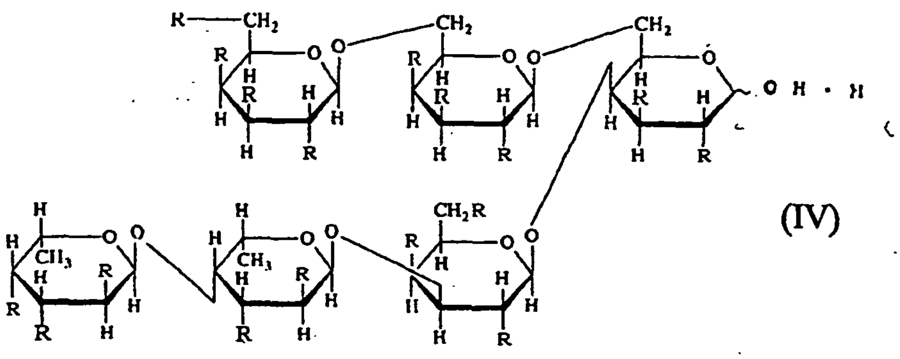

(IV)

wherein R is OH or $OSO_3H$.

Examples of the compound represented by the formula (I) include the compound represented by the formula (V) given later.

The compound represented by the formula (I) can be obtained by, for instance, treating the above-mentioned F-fucoidan with endo-sulfated polysaccharide degrading enzyme (F-fucoidan-specific degradation enzyme) produced by *Alteromonas sp.* SN-1009 (FERM BP-5747), and purifying the degradation product. As to the content and the site of sulfate group in the compound, any ones can be purified from the degradation product. In addition, the polymer of the compound represented by the formula (I) is contained in the degradation product, and can be separated and purified depending on its purposes.

Each of the compound represented by the formula (II) and the compound represented by the formula (III) can be obtained by, for instance, treating the above-mentioned U-fucoidan with endo-sulfated polysaccharide degrading enzyme (U-fucoidan-specific degradation enzyme) produced by *Flavobacterium sp.* SA-0082 (FERM BP-5402), and purifying the degradation product. As to the content and the site of sulfate group in the compound, any ones can be purified from the degradation product. In addition, the polymer of which basic backbone structure comprises the compound represented by the formula (II), but no double bonds, is also contained in the degradation product, and can be separated and purified depending on its purposes.

The compound represented by the formula (IV) can be obtained by, for instance, treating G-fucoidan with endo-sulfated polysaccharide degrading enzyme which specifically degrades G-fucoidan (G-fucoidan-specific degradation enzyme) produced by *Flavobacterium sp.* SA-0082 (FERM BP-5402) to give a degradation product of G-fucoidan, and purifying the degradation product. As to the content and the site of sulfate group in the compound, any ones can be purified from the degradation product. In addition, the polymer of which basic backbone structure comprises the compound

represented by the formula (IV) is also contained in the degradation product, and can be separated and purified depending on its purposes.

The action for regulation of cytokine production, induction of nitrogen monoxide production or anti-allergy for the fucoidan or a degradation product thereof can be examined, for instance, by the methods described in Examples 1, 2, 7 and 8 given below. In a case where the fucoidan or a degradation product thereof which is to be tested exhibits the above action, the fucoidan or a degradation product which can be used in the present invention is selected on the basis of the action.

In the present invention, the cytokine of which production can be regulated by the fucoidan or a degradation product thereof is not particularly limited. There are exemplified, for instance, interleukins (IL)-1 to -18, interferon (IFN)-α, IFN-β, IFN-γ, lymphotoxins, tumor necrosis factors (TNFs), stem cell factors (SCFs), granulocyte macrophage-colony stimulating factor (GM-CSF), granulocyte colony-stimulating factor (G-CSF), macrophage-colony stimulating factor (M-CSF), and the like.

These cytokines include those involved in expression and regulation of various cellular immune responses such as delayed-type hypersensitive reactions and target cytotoxicity (IL-2, IFN-γ, TNF-α, TNF-β and the like); those involved in the regulatory functions in the antibody production mechanism (IL-2, IL-4, IL-5, IL-6, and the like); those directly exhibiting suppressive action for proliferation or destroying action on tumor cells (TNF-α, TNF-β, IFN and the like); those capable of accelerating proliferation and differentiation of hematopoietic stem cells and precursor cells in the bone marrow (IL-1, IL-3, IL-4, IL-5, IL-6, IL-7, IL-9, IL-11, GM-CSF, G-CSF, M-CSF, and the like); those involved in inflammatory reactions (IL-1, IL-6, IL-8, TNF-α, TNF-β, IFN and the like); those involved in allergic reaction (IL-3, IL-4, IL-5 and the like); those capable of enhancing activity of NK cells (IL-12); and the like. The action for regulation of cytokine production owned by the fucoidan and/or a degradation product thereof used in the present invention refers to action for regulation of cytokine production which can be selectively induced only in a case where it is necessary for cellular immunity to be enhanced because of existence of an antigen to be removed, such as viral infection or cancer, for instance, action for induction of IFN-γ production or IL-12 production, or action for enhancement or acceleration of their production. Therefore, the fucoidan and/or a degradation product thereof does not cause activation of the immune system and enhancement thereof in an unnecessary state, so that they do not lead to a disease such as an allergic disease or autoimmune disease. Thus, the fucoidan and/or a degradation product thereof is a very safe and effective ingredient. In addition, the fucoidan and/or a degradation product thereof can bring about enhancement of cytotoxic immunity, for instance, via enhancement of the cytotoxic activity of lymphocytes.

Therefore, by using the fucoidan and/or a degradation product thereof to regulate the production of these cytokines, there can be treated or prevented a disease requiring regulation of cytokine production, such as a disease requiring expression and regulation of cellular immune response such as cancer; a disease requiring regulation of an antibody production such as autoimmune disease; a disease requiring differentiation of cells such as anemia or diabetes; or a disease requiring suppression of inflammation such as septic shock, inflammatory enteropathy, chronic articular rheumatism, multiple sclerosis, or uveitis.

The fucoidan and a degradation product thereof used in the present invention have an ability for regulation of cytokine production, so that a therapeutic agent or prophylactic agent for the above-mentioned disease requiring cytokine production can be prepared by using these compounds as effective ingredients.

In addition, the fucoidan and a degradation product thereof used in the present invention have action for induction of nitrogen monoxide production. By using these compounds as effective ingredients, there can be treated or prevented a disease requiring nitrogen monoxide production, such as arteriosclerosis requiring flaccidity of vascular smooth muscle, suppression of adhesion of thrombocytes, granulocytes and monocytes to vascular walls, inhibition of proliferation of secretory smooth muscle cells, and the like. The fucoidan and a degradation product thereof also exhibit an immunopotentiating effect through the above action. In addition, the action for induction of nitrogen monoxide production includes action for enhancement or acceleration of nitrogen monoxide production.

Further, the fucoidan and a degradation product thereof used in the present invention have anti-allergic action, such as action for suppression of IgE production. By using these compounds as effective ingredients, there can be treated or prevented an allergic disease, such as a symptom mediated or aggravated by IgE production, including, for instance, allergic diseases caused by IgE such as bronchial asthma, allergic rhinitis, atopic dermatitis, allergic conjunctivitis, urticaria, and anaphylactic shock.

A particular fractioned product of the above-mentioned fucoidan, especially F-fucoidan, U-fucoidan or G-fucoidan is a fucoidan-fractioned product of which basic structure has been clarified for the first time. Each degradation product prepared by using an enzyme specifically acting on each fractioned product, especially a fractioned product of the degradation product, such as compounds having the formulas (I) to (IV), has a low molecular weight, as compared to that of the fucoidan, and the same level of physiological activity as that of the fucoidan. This is why these fractioned products or degradation products are remarkably more excellent, as compared to a simply isolated fucoidan, of which structure, composition, or properties are indefinite, or the fucoidan-containing substance.

In the present invention, there is provided a therapeutic agent or prophylactic agent for a disease requiring

regulation of cytokine production, a disease requiring nitrogen monoxide production, or an allergic disease, comprising the fucoidan and/or a degradation product thereof as an effective ingredient.

Especially as the therapeutic agent or prophylactic agent for a disease requiring regulation of cytokine production of the present invention, from the viewpoint of efficacy, a therapeutic agent or prophylactic agent for a disease requiring regulation of interleukin or interferon production is preferable, and a therapeutic agent or prophylactic agent for a disease requiring regulation of interferon $\gamma$ or interleukin-12 production is more preferable. Also, the therapeutic agent or prophylactic agent for an allergic disease of the present invention is a very useful anti-allergic agent from the viewpoints of having action for suppression of IgE production after sensitization with an antigen, and suppression of IgE production after sensitization with an antigen in oral administration. Further, the therapeutic agent or prophylactic agent for an allergic disease of the present invention is remarkably excellent especially from the viewpoint that antigen-specific IgE production in a symptom sensitized with an antigen such as pollinosis can be suppressed in oral administration. Therefore, as the therapeutic agent or prophylactic agent for an allergic disease, a therapeutic agent or prophylactic agent for a disease requiring suppression of IgE production is preferable.

The therapeutic agent or prophylactic agent of the present invention comprises as an effective ingredient the fucoidan and/or a degradation product thereof, and is obtained by combining with a known medicinal vehicle to make a preparation. The preparation is generally produced by formulating the fucoidan and/or a degradation product thereof with a pharmaceutically acceptable liquid or solid vehicle, and optionally adding a solvent, a dispersant, an emulsifier, a buffer, a stabilizer, an excipient, a binder, a disintegrant, a lubricant, or the like, and forming the resulting mixture into a solid agent such as a tablet, a granule, a powder, a fine powder, or a capsule, or a liquid agent such as a general liquid agent, a suspension agent, or an emulsion agent. In addition, a dry product which can be made into a liquid form by adding an appropriate vehicle immediately before use can be also prepared.

The pharmaceutical vehicle can be properly selected depending upon the administration embodiment and the preparation form of the therapeutic agent or prophylactic agent of the present invention.

In the case of an orally administered preparation, for instance, starch, lactose, saccharose, mannitol, carboxymethyl cellulose, cornstarch, inorganic salts and the like are available. In addition, during the preparation of the orally administered preparation, a binder, a disintegrant, a surfactant, a lubricant, a fluidity accelerator, a flavor, a colorant, a perfume, or the like can be further formulated.

On the other hand, in the case of a non-orally administered preparation, according to the conventional method, the preparation can be produced by dissolving or suspending the fucoidan and/or a degradation product thereof, which is an effective ingredient of the present invention, in distilled water for injection, physiological saline, aqueous glucose solution, vegetable oil for injection, sesame oil, peanut oil, soybean oil, corn oil, propylene glycol, polyethylene glycol or the like as a diluent, and optionally adding a sterilizer, a stabilizer, an osmotic regulator, a soothing agent, or the like.

The therapeutic agent or prophylactic agent of the present invention can be administered via an administration route appropriate for each of the preparation form. The administration method is not limited to specific one. The agent can be administered internally or externally (or topically) or by injection. The injection can be administered, for instance, intravenously, intramuscularly, subcutaneously, intracutaneously, or the like. External preparations include a suppository. The therapeutic agent or prophylactic agent is preferably a therapeutic agent for oral administration or a prophylactic agent for oral administration, from the viewpoint of its efficacy.

The dose for the therapeutic agent or prophylactic agent of the present invention is changeable and properly set depending upon its preparation form, administration method, purpose of use, age, body weight, symptom or the like of the patient to which the agent is applied, or the like. The dose for adult per day is generally preferably from 0.1 to 2000 mg/kg as the amount of the fucoidan and/or a degradation product thereof contained in the preparation. Therefore, the content of the fucoidan and/or a degradation product thereof in the therapeutic agent or prophylactic agent of the present invention can be properly determined so that the fucoidan and/or a degradation product thereof can be administered at least in the above-mentioned range by administering the therapeutic agent or prophylactic agent. As a matter of course, the dose varies depending upon various conditions as mentioned above, so that an amount smaller than the dose mentioned above may be sufficient, or an amount exceeding the dose range may be required. The therapeutic agent or prophylactic agent of the present invention can be directly orally administered, or the agent can be added to any foodstuffs to take it on a daily basis. Also, the fucoidan and/or a degradation product thereof may be used as a raw material of foodstuffs or feed for regulation of cytokine production, induction of nitrogen monoxide production, or anti-allergy.

In addition, in one embodiment of the present invention, there is provided use of the fucoidan and/or a degradation product thereof for treating or preventing a disease requiring regulation of cytokine production, a disease requiring nitrogen monoxide production, or an allergic disease; or a method of treating or preventing a disease requiring regulation of cytokine production, a disease requiring nitrogen monoxide production, or an allergic disease, wherein the fucoidan and/or a degradation product thereof is used.

In order to treat or prevent a disease requiring regulation of cytokine production, a disease requiring nitrogen monoxide production, or an allergic disease, the fucoidan and/or a degradation product thereof may be, for instance,

administered in the range exemplified above. For instance, the therapeutic agent or prophylactic agent of the present invention is properly administered.

In still another embodiment of the present invention, there is provided an agent for regulation of cytokine production, such as an agent for regulation of IFN-γ or an agent for regulation of IL-12; an agent for induction of nitrogen monoxide production; or an anti-allergic agent, such as an agent for suppression of IgE production, each comprising as an effective ingredient the fucoidan and/or a degradation product thereof. These medicaments can be manufactured in the same manner as the above-mentioned therapeutic agent or prophylactic agent. For instance, the agent for regulation of cytokine production is useful for functional studies of cytokines, and screening of medicaments for a disease associated with cytokines. The preparation forms and the used amount of these medicaments are not particularly limited, as long as a desired effect exhibited by the medicament depending upon each use is obtained. These agents can be also applied to the diseases exemplified above.

In still another embodiment of the present invention, there is provided use of the fucoidan and/or a degradation product thereof for regulation of cytokine production, induction of nitrogen monoxide production, suppression of allergy, or suppression of IgE production; or a method for regulating cytokine production, inducing nitrogen monoxide production, suppressing allergy, or suppressing IgE production, wherein the fucoidan and/or a degradation product thereof is used.

In order to carry out regulation of cytokine production, induction of nitrogen monoxide production, suppression of allergy, or suppression of IgE production, for instance, the agent for regulation of cytokine production, induction of nitrogen monoxide production or the like may be used so as to obtain a desired effect depending upon each use.

The deduced pharmacological action for the fucoidan or a degradation product used in the present invention will be explained below.

As the induction of IFN-γ production from lymphocytes, there have been known induction via direct stimulation of T cells by mitogen such as Con A, and via intercellular interaction between antigen presenting cells (APCs) and T cells during sensitization with an antigen. The former is the induction by direct binding of the mitogen to T cell receptor, and the latter is the induction of production by stimulation of each receptor of APC and T cell, and action of IL-12 produced from APC by the stimulation. As the reaction for the receptor involved in induction of IL-12 production, there have been known each reaction, as side stimulation, of CD40L on the T cell side with CD40 on the APC side, and CD28 on the T cell side with B7 on the APC side in addition to the reaction of T cell receptor (TCR)/major histocompatibility complex (MHC).

Conventionally, there has been reported that a fucoidan derived from *Laminaria japonica* induces IFN-γ production from spleen lymphocytes derived from normal mouse to enhance the activation of NK cells (Chugoku Kaiyo Yakubutsu Zasshi (Zhongguo Haiyang Yaown, 1995, Third Period, 9-13). However, this is the induction caused by direct stimulation to naive T cells in a resting phase.

On the other hand, regarding the action for induction of IFN-γ production of the fucoidan derived from *Kjellmaniella crassifolia* and a fraction thereof presently confirmed by the present inventors, as shown in Examples, the induction action was not found in the direct stimulation or the alloantigen stimulation of lymphocytes, and the IFN-γ production was increased only under the antigen stimulation of sensitized lymphocytes. Further, in this case, the induction of IL-12 production was also found. The action for induction of IFN-γ production during this antigen-presenting reaction was such that about 50% of the action is suppressed when IL-12 produced is neutralized by an anti-IL-12 antibody, and that the action is completely suppressed when the reaction of CD40 and CD28 with their receptors is inhibited by an anti-CD40 antibody and an anti-CD28 antibody. From this fact, it is deduced on the action mechanism for the induction of IFN-γ production of the fucoidan derived from *Kjellmaniella crassifolia* and a fraction thereof that the induction of IFN-γ results from the enhancement of IL-12 production from APC by the action of the fucoidan and a fraction thereof to APC during antigen-presenting reaction, and from the action of the fucoidan and a fraction thereof to T cell activated by the interaction of APC and T cell.

The fucoidan described in the above-mentioned report which studies on a direct induction action of the fucoidan on T cell in a resting phase is different from the fucoidan derived from *Kjellmaniella crassifolia* used in the present invention. Therefore, their fucoidans exhibit completely different action, respectively.

In addition, it was also shown in a system using a macrophage cell line, one of antigen presenting cells, that the fucoidan and a degradation product thereof induces nitrogen monoxide production, and exhibits the immunopotentiating effect through the induction. However, even in this system, there was no induction of IFN-γ production by the fucoidan and a degradation product thereof. It is considered that the fucoidan and a degradation product thereof used in the present invention does not have activity for direct induction of IFN-γ production, and that nitrogen monoxide production are enhanced parallel to IL-12 production. From the above, there can be deduced that the enhancement of IFN-γ production by the fucoidan and a degradation product thereof according to the present invention is caused via the enhancement of IL-12 production from APC by the action of the fucoidan and a degradation product thereof to APC during antigen presenting reaction, not by the induction of IFN-γ production by direct stimulation of T-cells in a resting phase, and that the enhancement of IFN-γ production is induced by the action of the fucoidan and a degradation thereof to T cell activated by the interaction of APC and T cell. From this aspect, the fucoidan and a degradation product thereof

used in the present invention exhibit completely different action from that of the above-mentioned report which studies on the direct induction action.

Here, IFN-γ is produced when a T cell receptor of T cell (Th1) is stimulated with antigen presenting cell (APC) and the like, and the IFN-γ production is enhanced by IL-12 produced from APC. IFN-γ activates cellular immunity involving NK cell, macrophage, and the like in a case such as viral infection, mycosis, and cancer diseases, thereby acting to enhance biophylaxis ability.

On the other hand, IFN-γ has been known to suppress the activation of Th2 cells which are considered to be causative of generation of allergic diseases represented by asthma, pollinosis, atopic dermatitis, and the like. Th2 cells induce production of immunoglobulin E antibody (IgE). The produced IgE is bound to a receptor on a cell membrane of a mast cell, thereby causing release of a chemical mediator from the mast cell. The allergic symptom is developed due to this inflammatory mediator as a direct causation.

The anti-allergic agent of the present invention suppresses IgE production in the oral administration after sensitization with an antigen, thereby being very useful in ameliorations of symptoms in allergic diseases represented by asthma, pollinosis, atopic dermatitis, and the like after onset.

Further, the present invention provides a food or beverage for regulation of cytokine production, induction of nitrogen monoxide production, or anti-allergy, wherein the food or beverage comprises the fucoidan and/or a degradation product thereof.

Especially as the food or beverage for regulation of cytrokine production of the present invention, from the viewpoint of efficacy, a food or beverage for regulation of interleukin production or interferon production is preferable, and a food or beverage for regulation of interferon γ production or interleukin-12 production is more preferable.

Also, the food or beverage for anti-allergy of the present invention is a very useful foodstuff from the viewpoints of having action for suppression of IgE production after sensitization with an antigen, and exhibiting action for suppression of IgE production after sensitization with an antigen by its intake. Therefore, as the food or beverage for anti-allergy, a food or beverage for suppression of IgE production is preferable.

As to the feed described later, from the same viewpoints as above, the feed having the above action is preferable.

Since the food or beverage has action for regulation of cytokine production, induction of nitrogen monoxide production, or anti-allergy, the food or beverage is very useful in amelioration or prevention of symptoms for a disease requiring regulation for cytokine production, a disease requiring nitrogen monoxide production, or an allergic disease, which is sensitive to the fucoidan or a degradation product thereof.

The term "comprise or comprising" as referred to in the food, beverage or feed, or the cosmetic (described below) of the present invention includes the meanings of containing, adding and diluting. The term "containing" refers to an embodiment of containing the effective ingredient used in the present invention in the food, beverage or the like; the term "adding" refers to an embodiment of adding the effective ingredient used in the present invention to a raw material for the food, beverage or the like; and the term "diluting" refers to an embodiment of adding a raw material for the food, beverage or the like to the effective ingredient used in the present invention.

The method for manufacturing the food or beverage of the present invention is not particularly limited. The food or beverage may be manufactured according to a known method. For instance, the method includes, cooking, processing, and any methods for manufacturing food or beverage generally employed, as long as the resulting food or beverage contains as an effective ingredient the fucoidan and/or a degradation product thereof, wherein the fucoidan and or a degradation product thereof has action for regulation of cytokine production, induction of nitrogen monoxide production or anti-allergy.

The food or beverage of the present invention is not particularly limited. The food or beverage includes those comprising the fucoidan and/or a degradation product as an effective ingredient, including, for instance, processed agricultural and forest products, processed stock raising products, processed marine products and the like, including processed grain products such as processed wheat products, processed starch products, processed premix products, noodles, macaronis, bread, bean jam, buckwheat noodles, wheat-gluten bread, rice noodle, *fen-tiao*, and packed rice cake; processed fat and oil products such as plastic fat and oil, tempura oil, salad oil, mayonnaise, and dressing; processed soybean products such as tofu products, soybean paste, and fermented soybeans; processed meat products such as ham, bacon, pressed ham, and sausage; marine products such as frozen ground fish, boiled fish paste, tubular roll of boiled fish paste, cake of ground fish, deep-fried patty of fish paste, fish ball, sinew, fish meat ham and sausage, dried bonito, products of processed fish egg, marine cans, and preserved food boiled down in soy sauce (*tsukudani*); milk products such as raw material milk, cream, yogurt, butter, cheese, condensed milk, powder milk, and ice cream; processed vegetable and fruit products such as paste, jam, pickled vegetables, fruit beverages, vegetable beverages, and mixed beverages; confectionaries such as chocolates, biscuits, sweet bun, cake, rice cake snacks, and rice snacks; alcohol beverages such as *sake,* Chinese liquor, wine, whisky, Japanese distilled liquor (*shochu*), vodka, brandy, gin, ram, beer, refreshing alcoholic beverages, fruit liquor, and liqueur; luxury drinks such as green tea, tea, oolong tea, coffee, refreshing beverages and lactic acid beverages; seasonings such as soy sauce, sauce, vinegar, and sweet rice wine; canned, binned or pouched foods such as rice topped cooked beef and vegetable, rice boiled together with meat

and vegetables in a small pot, steamed rice with red beans, curry roux and rice, and other precooked foods; semi-dry or concentrated foods such as liver pastes and other spreads, soups for buckwheat noodles or wheat noodles, and concentrated soups; dry foods such as instant noodles, instant curry roux, instant coffee, powder juice, powder soup, instant soybean paste (*miso*) soup, precooked foods, precooked beverages, and precooked soup; frozen foods such as *sukiyaki,* pot-steamed hotchpotch, split and grilled eel, hamburger steak, *shao-mai*, dumpling stuffed with minced pork, various sticks, and fruit cocktails; solid foods; liquid foods (soups); spices; and the like.

The food or beverage of the present invention comprises the fucoidan and/or a degradation product thereof having physiological action selected from action for regulation of cytokine production, action for induction of nitrogen monoxide production, anti-allergic action and the like, and its shape is not particularly limited as long as an amount necessary for the fucoidan and/or a degradation product thereof to exhibit the physiological action is contained. The food or beverage including, for instance, products shaped into tablets, granules, capsules or the like, which can be orally taken.

The content of the fucoidan and/or a degradation product in the food or beverage can be properly selected on the basis of sensory and physiological activity. The content is, for instance, $10^{-9}$ parts by weight or more, preferably from $10^{-7}$ to 2 parts by weight, per 100 parts by weight of the food, or for instance, $10^{-9}$ parts by weight or more, preferably from $10^{-7}$ to 2 parts by weight, per 100 parts by weight of the beverage. For instance, the food or beverage may be taken such that the fucoidan and/or a degradation product thereof can be taken in an amount of from 0.01 to 2000 mg/kg per day for adult.

Here, the fucoidan and a degradation product thereof having physiological action selected from action for regulation of cytokine production, action for induction of nitrogen monoxide production, anti-allergic action and the like are extremely useful production material for a food or beverage as a health food material having both physiological action and dietary fiber function.

In addition, according to the present invention, there is provided a feed for an organism comprising as an effective ingredient the fucoidan and/or a degradation product thereof having physiological action selected from action for regulation of cytokine production, action for induction of nitrogen monoxide production, anti-allergic action and the like.

Also in one embodiment of the present invention, there is provided a method of feeding an organism, comprising administering the fucoidan and/or a degradation product thereof having the above physiological action to the organism.

Also in one embodiment of the present invention, there is provided an organism-feeding agent comprising the fucoidan and/or a degradation product thereof having the above physiological action.

In these inventions, the organism includes, for instance, culturing or breeding animals, pet animals, and the like. The culturing or breeding animal is exemplified by cattle, experimental animals, poultry, pisces, crustaceae or shellfish.

The feed is exemplified by a feed for physical condition improvement on the basis of the physiological action of the fucoidan and/or a degradation product thereof.

The organism-feeding agent includes immersion agents, feed additives, and beverage additives.

In these inventions, the fucoidan and/or a degradation product thereof having the above physiological action has an effect of improving a feeding efficiency of an organism, for instance, survival rate, fattening ratio, egg production ratio, calf production ratio, weaning ratio, or the like.

In the method of feeding an organism comprising administering the fucoidan and/or a degradation product thereof to an organism, the fucoidan and/or a degradation product thereof having the above physiological action may be usually administered in an amount of preferably from 0.01 to 2000 mg per 1 kg of body weight of the subject organism per day. The administration can be carried out by, for instance, adding and mixing the fucoidan and/or a degradation product thereof in a raw material for an artificially formulated feed, or mixing the fucoidan and/or a degradation product thereof with a powder raw material for an artificially formulated feed, and thereafter further adding and mixing other raw materials with the resulting mixture. In addition, the feed of the present invention may be administered so that the above dose of the fucoidan and/or a degradation product thereof can be achieved.

The content of the fucoidan and/or a degradation product thereof having the above physiological action in the feed is not particularly limited. The fucoidan and/or a degradation product thereof may be used in accordance with its purposes, and a preferable proportion in the feed is from 0.001 to 15% by weight.

The artificially formulated feed includes artificially formulated feeds comprising as raw materials animal-derived raw materials such as fish meal, casein, and squid meal; plant-derived raw materials such as soybean grounds, flour, starch and yeasts for feed; animal fats and oils such as cod-liver oil and squid-liver oil; vegetable fats and oils such as soybean oil and rapeseed oil; vitamins, minerals, amino acids, and antioxidants; and the like. In addition, feeds for fish such as fish minced meat are also included.

In addition, functional food materials including the functional oligosaccharides such as fructooligosaccharide and isomaltooligosaccharide; dietary fibers such as polydextrose, hardly digestible dextrin, and β-1,3-glucan; propolis, sugar alcohols such as maltitol, palatinit, and erythritol; EPA, γ-linolenic acid, heme, chlorella extracts, *Spirulina* extracts, and whiteberry extracts; cariostatic materials such as polyphenols may be used as a raw material for the feed of the

present invention. Here, as the feed in the present invention, there can be encompassed a feed additive and a nutritional supplement for a feed.

The method for manufacturing the feed of the present invention is not particularly limited, as long as an effective amount of the fucoidan and/or a degradation product thereof having physiological action selected from action for regulation of cytokine production, induction of nitrogen monoxide production, anti-allergy, and the like is contained in the feed manufactured.

Also, the fucoidan and/or a degradation product thereof having the above physiological action can be administered by directly adding the fucoidan and/or a degradation product thereof to water, seawater, or the like in a pool, a water tank, a water reservoir, or a feeding range, and immersing a subject organism into the resulting solution. The immersion method is effective especially when the amount of intake of the feed of the subject organism is lowered.

The concentration of the fucoidan and/or a degradation product thereof in water or seawater is not particularly limited, and the fucoidan and/or a degradation product thereof may be used in accordance with its purposes. It is preferable that the concentration is from 0.00001 to 1% by weight.

Also, a beverage comprising the fucoidan and/or a degradation product thereof having the above physiological action may be given to a subject organism as a feeding drink.

The concentration of the fucoidan and/or a degradation product thereof having the above physiological action in the beverage is not particularly limited, and the fucoidan and/or a degradation product thereof may be used in accordance with its purposes. It is preferable that the concentration is from 0.0001 to 1% by weight.

The organism feeding agent, for instance, an immersion agent, a feed additive, or a beverage additive comprising as an effective ingredient the fucoidan and/or a degradation product thereof having the above physiological action may be prepared by a known method.

The organism to which the present invention can be applied is not limited. The culturing or breeding animals include cattle such as *Equus, Bos, Porcus, Ovis, Capra, Camelus,* and *Lama;* experimental animals such as mice, rats, guinea pigs, and rabbits; poultry such as *Chrysolophus,* ducks, *Meleagris,* and *Struthioniformes;* pisces such as *Pagrus, Oplegnathidae, Paralichthys,* plaice, *Seriola,* young *Seriola,* amberjack, *Thunna, Caranx delicatissimus, Plecoglossus, Salmo •Oncorhynchus, Fugu, Anguilla, Misguirus,* and *Parasilurus;* Crustaceae such as *Penaidae,* black tiger shrimp, *Penaeus roentalis,* and *Portulus trituberculatus;* and shellfish such as abalones (*awabi*), turban shells, scallops, and oysters; and the pet animals includes dogs, cats, and the like, so that the feed can be widely applied to animals on land and in water.

By allowing a subject organism to take the feed comprising the fucoidan and/or a degradation product thereof having physiological action selected from action for regulation of cytokine production, action for induction of nitrogen monoxide production, anti-allergic action and the like, or immersing a subject organism into a solution containing the fucoidan and/or a degradation product thereof, the physical conditions, health, biological homeostasis, metabolism and the like of cattle, experimental animals, poultry, pisces, Caustacea, shellfish, pet animals or the like are improved, so that the present invention is very useful in anti-aging prevention of an organism.

Further, the fucoidan and/or a degradation product thereof having physiological action selected from action for regulation of cytokine production, action for induction of nitrogen monoxide production, anti-allergic action, and the like is useful as an effective ingredient for a cosmetic. In one embodiment of the present invention, there is provided a cosmetic for regulation of cytokine production, induction of nitrogen monoxide production, anti-allergy, or the like, wherein the cosmetic comprises as an effective ingredient the fucoidan and/or a degradation product thereof.

The content of the fucoidan and/or a degradation product thereof contained in these cosmetics or the like is usually preferably from 0.0001 to 20% by weight, more preferably from 0.001 to 5% by weight.

The cosmetic of the present invention is effective for oral administration as well as percutaneous application. According to the present invention, there is provided a cosmetic effective for percutaneous application or oral administration. Especially the cosmetic for anti-allergy is useful for treatment or prevention of atopic dermatitis due to its anti-atopic action. Here, the amount of dose and the amount of application may be properly adjusted so as to obtain desired effects.

The cosmetic of the present invention can be manufactured in accordance with a conventional method. As the cosmetic for regulation of cytokine production, induction of nitrogen monoxide production or anti-allergy, there can be manufactured, for instance, a lotion, a milky lotion, cream, a facial pack, a bathing agent, a facial cleansing agent, a bathing soap, a hair tonic, a hair restorer, or a shampoo.

In addition, no case of death is found even when the fucoidan and/or a degradation product thereof having action for regulation of cytokine production, induction of nitrogen monoxide production, or anti-allergy used in the present invention is orally administered to a rat in a single dose of 1 g/kg.

## EXAMPLES

The present invention will be more concretely described by means of the examples, without limiting the scope

of the present invention thereto. Here, "%" in Examples means "% by weight."

Reference Example 1

(1) *Kjellmaniella crassifolia* was sufficiently dried, and thereafter 20 kg of the dried product was powdered with a free mill (manufactured by Nara Kikai Seisakusho).

In 900 liters of tap water was dissolved 7.3 kg of calcium chloride dihydrate (manufactured by Nippon Soda Co., Ltd.), and 20 kg of the powdered product of *Kjellmaniella crassifolia* was then mixed therewith. The resulting mixture was heated for 40 minutes until the liquid temperature was raised from 12°C to 90°C by blowing steam. Thereafter, the mixture was kept at 90° to 95°C for 1 hour under stirring, and then cooled, to give 1100 liters of a cooled product. Subsequently, the cooled product was subjected to solid-liquid separation with a solid-liquid separator (manufactured by West Farrier Separator, Model: CNA), to give about 900 liters of supernatant after solid-liquid separation.

The amount 360 liters of the supernatant after solid-liquid separation was concentrated up to a volume of 20 liters with FE10-FC-FUS0382 (fraction molecular weight: 30000) manufactured by DAICEL CHEMICAL INDUSTRIES, LTD. Thereafter, the steps of adding 20 liters of tap water and again concentrating the resulting liquid mixture up to a volume of 20 liters were repeated 5 times, and the concentrate was subjected to a desalting treatment, to give 25 liters of an extract derived from *Kjellmaniella crassifolia.*

One liter of the extract was lyophilized, to give 13 g of a dried product of fucoidan derived from *Kjellmaniella crassifolia*.

(2) Seven grams of the dried product of fucoidan described in item (1) of Reference Example 1 was dissolved in 700 ml of a 20 mM imidazole buffer (pH 8.0) containing 50 mM sodium chloride and 10% ethanol, and insoluble matters were removed by centrifugation. The supernatant after centrifugation was applied onto a DEAE-Cellulofine A-800 column (ϕ 11.4 cm x 48 cm) equilibrated with the same buffer, and then washed with the same buffer. The elution was carried out with a concentration gradient of from 50 mM to 1.95 M sodium chloride (250 ml per fraction). A total sugar content and an uronic acid content were determined by the phenol-sulfuric acid method and the carbazole-sulfuric acid method, to give Fractions 43 to 49, Fractions 50 to 55, and Fractions 56 to 67, in the order of elution. Next, these fractions were desalted by electrodialysis, and thereafter lyophilized, to give each of Fraction I (340 mg) from Fractions 43 to 49, Fraction II (870 mg) from Fractions 50 to 55, and Fraction III (2.64 g) from Fractions 56 to 67.

Figure 1 shows an elution pattern of the fucoidan derived from *Kjellmaniella crassifolia* on the DEAE-Cellulofine A-800 column. In Figure 1, the axis of ordinates is the absorbance at 530 nm as determined by the carbazole-sulfuric acid method (solid circles in the figure), the absorbance at 480 nm as determined by the phenol-sulfuric acid method (open circles in the figure), and the electric conductivity (mS/cm: open squares in the figure), and the axis of abscissas is the fraction number.

Reference Example 2

(1) A 2-liter Erlenmeyer flask was charged with 600 ml of a culture medium comprising an artificial sea water (manufactured by Jamarin Laboratory), pH 8.2, containing 0.25% glucose, 1.0% peptone, and 0.05% yeast extract, and then sterilized (at 120°C for 20 minutes). *Alteromonas* sp. SN-1009 (FERM BP-5747) was inoculated into the culture medium, and cultured at 25°C for 26 hours, to give a seed culture medium. A 30-liter jar fermentor was charged with 20 liters of a culture medium comprising an artificial sea water, pH 8.0, containing 1.0% peptone, 0.02% yeast extract, 0.2% sulfated polysaccharide described in item (2) of Reference Example 2 described below, and 0.01% defoaming agent (manufactured by Shin-Etsu Chemical Co., Ltd., KM70), and sterilized at 120°C for 20 minutes. After cooling, 600 ml of the above-mentioned seed culture medium was inoculated, and cultured at 24°C for 24 hours under the conditions of 10 liters of aeration per minute and a stirring rate of 250 rpm. After termination of the culture, the culture medium was centrifuged, to give cells and culture supernatant. The culture supernatant obtained was concentrated with an ultrafilter equipped with holofiber having an excluding molecular weight of 10000, and the concentrate was then subjected to salting out with an 85% saturated ammonium sulfate. Precipitates formed were harvested by centrifugation, and sufficiently dialyzed against a 20 mM Tris-HCl buffer (pH 8.2) containing an artificial sea water at a one-tenth concentration, to give 600 ml of a solution of an endo-sulfated polysaccharide-degrading enzyme (F-fucoidan-specific degradation enzyme), selectively acting on the sulfated polysaccharide.

(2) Two kilograms of dried *Kjellmaniella crassifolia* was powdered with a cutter mill (manufactured by Masuko Sangyo) fitted with a screen having a diameter of 1 mm, and the resulting seaweed chips were suspended in 20

liters of 80% ethanol. The suspension was stirred at 25°C for 3 hours and filtered with a filter paper, and thereafter the residue was sufficiently washed. The residue obtained was suspended in 40 liters of a 20 mM sodium phosphate buffer, pH 6.5, which was heated to 95°C, the buffer containing 50 mM sodium chloride. The suspension was treated at 95°C for 2 hours with occasional stirring, to extract a sulfated polysaccharide.

The suspension of the extract was filtered, to give a filtrate. Thereafter, the filtration residue was washed with 3.5 liters of 100 mM sodium chloride, to give an additional filtrate.

Both filtrates were combined, and then the temperature was lowered to 30°C. After 3000 U of alginic acid lyase K (manufactured by Nagase Seikagaku Kogyo) was added to the resulting mixture, 4 liters of ethanol was added thereto. The resulting mixture was stirred at 25°C for 24 hours. Next, the mixture was centrifuged, and the resulting supernatant was concentrated up to a volume of 4 liters with an ultrafilter equipped with holofiber having an excluding molecular weight of 100000. Further, the ultrafiltration was continued with 100 mM sodium chloride containing 10% ethanol until a colored substance was no longer filtered.

Precipitates formed in a non-filtrate solution were removed by centrifugation, and the temperature of the resulting supernatant was lowered to 5°C. The pH was adjusted to 2.0 with 0.5 N hydrochloric acid, and thereafter the formed precipitates such as a protein were removed by centrifugation. The pH of the resulting supernatant was rapidly adjusted to 8.0 with 1 N sodium hydroxide.

Next, an ultrafiltration was carried out with an ultrafilter equipped with holofiber having an excluding molecular weight of 100000, and the solvent was completely substituted with 20 mM sodium chloride, pH 8.0. Thereafter, the pH was again adjusted to 8.0, and the resulting mixture was centrifuged and then lyophilized, to give about 95 g of a sulfated polysaccharide.

(3) Two kilograms of dried *Kjellmaniella crassifolia* was powdered with a cutter mill fitted with a screen having a diameter of 1 mm, and the resulting seaweed chips were suspended in 20 liters of 80% ethanol. The resulting suspension was stirred at 25°C for 3 hours, and filtered with a filter paper, and thereafter the residue was sufficiently washed. The residue obtained was suspended in 20 liters of a buffer (pH 8.2) containing 30 ml of a solution of the endo-sulfated polysaccharide-degrading enzyme prepared in item (1) of the above-mentioned Reference Example 2, 10% ethanol, 100 mM sodium chloride, 50 mM calcium chloride and 50 mM imidazole, and the resulting mixture was stirred at 25°C for 48 hours. This suspension was filtered with a stainless screen having a screen-opening diameter of 32 μm, and the residue was washed with 10% ethanol containing 50 mM calcium chloride. Further, the residue was suspended in 10 liters of 10% ethanol containing 50 mM calcium chloride, and the suspension was stirred for 3 hours, and thereafter filtered with the stainless screen, and the residue was washed. Further, the residue was suspended under the same conditions, and the suspension was then stirred for 16 hours. The suspension was filtered with the stainless screen having a diameter of 32 μm, and the residue was washed.

The filtrate and the washings thus obtained were collected, and the combined mixture was subjected to ultrafiltration with an ultrafilter equipped with holofiber having an excluding molecular weight of 3000, thereby separating a filtered solution from a non-filtered solution.

This filtered solution was concentrated to a volume of about 3 liters with a rotary evaporator, and thereafter the concentrate was centrifuged, to give supernatant. The supernatant obtained was desalted with an electric dialyzer equipped with a membrane having an excluding molecular weight of 300. To the resulting solution was added calcium acetate so as to give a concentration of 0.1 M, and precipitates formed were removed by centrifugation. The resulting supernatant was applied onto a DEAE-Cellulofine column (amount of resin: 4 liters) previously equilibrated with 50 mM calcium acetate, and sufficiently washed with 50 mM calcium acetate and 50 mM sodium chloride. Thereafter, the elution was carried out with a concentration gradient of from 50 mM to 800 mM sodium chloride. The amount collected at this time was 500 ml per fraction. The collected fraction was analyzed by cellulose acetate membrane electrophoresis [Analytical Biochemistry, 37, 197-202 (1970)]. As a result, a sulfated saccharide which was eluted on a concentration of about 0.4 M sodium chloride (Proximity of Fraction No. 63) was homogeneous.

Then, a solution of Fraction No. 63 was first concentrated to a volume of 150 ml, and thereafter sodium chloride was added so as to give a concentration of 4 M. The resulting solution was applied onto a Phenyl-Cellulofine column (amount of resin: 200 ml) previously equilibrated with 4 M sodium chloride, and sufficiently washed with 4 M sodium chloride. Non-adsorptive sulfated saccharide fractions were collected, and desalted with an electrodialyzer equipped with a membrane having an excluding molecular weight of 300, to give 505 ml of a desalted solution.

Forty milliliters of the desalted solution obtained was applied onto a Cellulofine GCL-90 column (4.1 cm x 87 cm) equilibrated with 0.2 M sodium chloride containing 10% ethanol, to perform gel filtration. The collection was performed at 9.2 ml per fraction.

All of the fractions were analyzed for a total sugar content by the phenol-sulfuric acid method [Analytical Chemistry, 28, 350 (1956)].

As a result, since the sulfated saccharide formed a single peak, Fraction Nos. 63 to 70, which were fractions corresponding to a central part of the peak were collected. The combined fraction was desalted with an electrodialyzer

equipped with a membrane having an excluding molecular weight of 300, and thereafter lyophilized, to give 112 mg of a dried product of the compound represented by the following formula V.

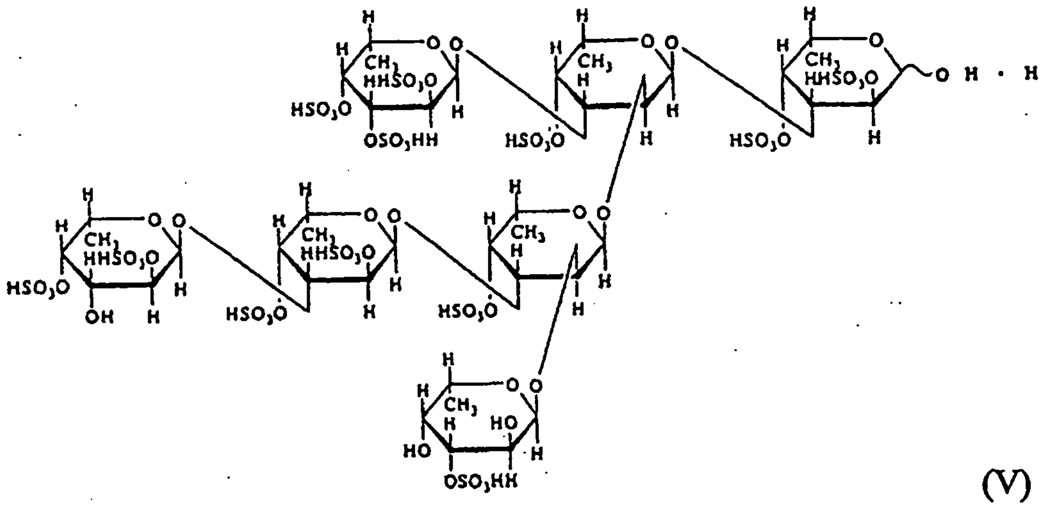

(V)

Reference Example 3

(1) Two kilograms of dried *Kjellmaniella crassifolia* was powdered with a cutter mill (manufactured by Masuko Sangyo) fitted with a screen having a hole diameter of 1 mm. After the powdered product was stirred in 20 liters of 80% ethanol at 25°C for 3 hours, the mixture was filtered, and the residue was washed. The resulting residue was suspended in 20 liters of a 30 mM imidazole buffer (pH 8.2) containing 50 mM calcium chloride, 100 mM sodium chloride, 10% ethanol, and 1 U of *Alteromonas* sp. SN-1009 (FERM BP-5747) endo-sulfated polysaccharide-degrading enzyme (F-fucoidan-specific degradation enzyme) prepared in item (1) of Reference Example 2. The resulting suspension was stirred at 25°C for 2 days, and thereafter filtered with a stainless screen having a hole diameter of 32 μm, and the residue was washed. The resulting residue was suspended in 40 liters of a sodium phosphate buffer (pH 6.6) containing 100 mM sodium chloride, 10% ethanol and 4 g of an alginic acid lyase (manufactured by Nagase Seikagaku Kogyo). The resulting suspension was stirred at 25°C for 4 days, and thereafter centrifuged, to give supernatant. In order to remove low-molecular weight products of alginic acid contained in the supernatant obtained, the supernatant was concentrated to a volume of 2 liters with an ultrafilter equipped with holofiber having an excluding molecular weight of 100000, and thereafter the solvent was exchanged for 100 mM sodium chloride containing 10% ethanol. To the resulting solution was added with stirring an equivolume of 400 mM calcium acetate, and thereafter the mixture was centrifuged. The pH of the resulting supernatant was adjusted to 2 with 1 N hydrochloric acid, with cooling on ice. Precipitates formed were removed by centrifugation, and the pH of the resulting supernatant was adjusted to 8.0 with 1 N sodium hydroxide. This solution was concentrated to a volume of 1 liter by ultrafiltration, and thereafter the solvent was exchanged for 100 mM sodium chloride. Precipitates formed at this time were removed by centrifugation. In order to remove hydrophobic substances in the resulting supernatant, sodium chloride was added to the supernatant so as to give a concentration of 1 M, and the resulting mixture was applied onto a column containing 3 liters of Phenyl-Cellulofine (manufactured by Seikagaku Corporation) equilibrated with 1 M sodium chloride, to collect an effluent fraction. The fraction was concentrated with an ultrafilter, and thereafter the solvent was exchanged for 20 mM sodium chloride. The resulting solution was lyophilized, and the weight of the lyophilized product was 29.3 g.

(2) Fifteen grams of the above-mentioned lyophilized product was dissolved in 1.5 liters of 50 mM Tris-HCl buffer containing 400 mM sodium chloride and 9 U of an endo-sulfated polysaccharide-degrading enzyme (U-fucoidan-specific degradation enzyme) obtained from a culture in the same manner as in item (1) of Reference Example 2, the culture prepared by culturing *Flavobacterium* sp. SA-0082 (FERM BP-5402) disclosed in WO97/26896. After

the resulting solution was subjected to the reaction at 25°C for 6 days, the reaction mixture was concentrated to a volume of about 300 ml with an evaporator. The concentrate was placed in a dialyzing tube having an excluding molecular weight of 3500 and thoroughly dialyzed. The solution remaining in the dialysis tube was applied onto a column containing 4 liters of DEAE-Cellulofine A-800 equilibrated with 50 mM sodium chloride, and sufficiently washed with 50 mM sodium chloride. Thereafter, the elution was carried out on a concentration gradient of from 50 to 650 mM sodium chloride. Further, the elution was sufficiently carried out in the same column with 650 mM sodium chloride. Among the eluted fractions, the fractions eluted with 650 mM sodium chloride were collected as a sulfated fucogalactan fraction, and concentrated with an ultrafilter having an excluding molecular weight of 100000. Thereafter, the solvent was substituted with 10 mM sodium chloride, and the resulting solution was lyophilized, to give 0.85 g of a lyophilized product of sulfated fucogalactan. The sulfated fucogalactan obtained (G-fucoidan) was found to contain galactose and fucose as constituting saccharides in a molar ratio of about 2:1.

Reference Example 4

One kilogram of a dried product of a commercially available sporophyll of *Undaria pinnatifida* (Wakame Mekabu) was powdered with a cutter mill fitted with a screen having a hole diameter of 1 mm. Thereafter, the powdered sporophyll was suspended in 10 liters of 80% ethanol, and the suspension was stirred for 3 hours, and thereafter filtered with a filter paper, to give a residue. The residue was suspended in 20 liters of a 40 mM sodium phosphate buffer (pH 6.5) containing 50 mM sodium chloride, and treated at 95°C for 2 hours. The treated solution was cooled to 37°C, and thereafter ethanol was added thereto so as to give a concentration of 10%. 12000 U of a commercially available alginic acid lyase K (manufactured by Nagase Seikagaku Kogyo) was added thereto, and thereafter the mixture was stirred at room temperature for 24 hours. The resulting treated solution was centrifuged, and the resulting supernatant was concentrated to a volume of 2 liters with an ultrafilter equipped with holofiber having an excluding molecular weight of 100000. Thereafter, precipitates formed were removed by centrifugation. The resulting supernatant was cooled to 5°C, and thereafter 0.5 N hydrochloric acid was added thereto to adjust the pH to 2.0. Subsequently, the resulting mixture was stirred for 30 minutes, and precipitates formed were removed by centrifugation. The pH of the resulting supernatant was adjusted to 8.0 with 0.5 N sodium hydroxide, and the solvent was substituted with 20 mM sodium chloride by ultrafiltration. The pH of the resulting solution was adjusted to 8.0, and thereafter the supernatant obtained by centrifugation was lyophilized, to give 90.5 g of fucoidan derived from sporophyll of *Undaria pinnatifida*.

Reference Example 5

One kilogram of a dried product of powdered *Fucus vesiculosus* was suspended in 10 liters of 80% ethanol, and the suspension was stirred for 3 hours, and thereafter filtered with a filter paper, to give a residue. The residue was suspended in 30 liters of a 30 mM sodium phosphate buffer (pH 6.0) containing 100 mM sodium chloride, and treated at 95°C for 2 hours. After the treated solution was cooled to 37°C, 100 g of activated carbon was added, and the mixture was stirred for 30 minutes. After 3000 U of a commercially available alginic acid lyase K was added, ethanol was added so as to give a concentration of 10%, and the resulting mixture was stirred at room temperature for 24 hours. The resulting treated solution was centrifuged, and the supernatant was concentrated to a volume of 2 liters with an ultrafilter equipped with holofiber having an excluding molecular weight of 100000. Thereafter, precipitates formed were removed by centrifugation, and the resulting supernatant was ultrafiltered with an extract added, to remove a pigment. The non-filtered solution obtained was cooled to 5°C, and thereafter 0.5 N hydrochloric acid was added thereto to adjust the pH to 2.0. Thereafter, the resulting solution was stirred for 30 minutes, and precipitates formed were removed by centrifugation. The pH of the supernatant was adjusted to 8.0 with 0.5 N sodium hydroxide, and the solvent was substituted with 20 mM sodium chloride by ultrafiltration. The pH of the resulting solution was adjusted to 8.0, and thereafter the supernatant obtained by centrifugation was lyophilized, to give 71 g of fucoidan derived from *Fucus vesiculosus.*

Reference Example 6

Two grams of fucoidan derived from *Kjellmaniella crassifolia* prepared by the method described in item (1) of Reference Example 1 was dissolved in 100 ml of water, and the pH of the solution was adjusted to pH 3 with citric acid. Thereafter, the resulting mixture was treated at 100°C for 3 hours, to give a hydrolysate with the acid of the fucoidan. This hydrolysate was subjected to molecular weight fractionation by gel filtration on Cellulofine GCL-300 or Cellulofine GCL-25, to fractionate the hydrolysate into fractions exceeding MW 25000 (Fraction A); exceeding MW 10000 to MW 25000 (Fraction B); exceeding MW 5000 to 10000 (Fraction C); exceeding MW 2000 to 5000 (Fraction D); exceeding MW 500 to 2000 (Fraction E); and MW 500 or less (Fraction F). Further, each of these fractions and the hydrolysate were desalted, and then lyophilized, to give the hydrolysate and each fraction of the hydrolysate.

Reference Example 7

Five kilograms of sea cucumbers were dissected, and the organs were removed to collect somatic layers. Five-hundred milliliters of acetone was added per 200 g of the wet weight of the somatic layers, and the mixture was treated with a homogenizer. Thereafter, the homogenate was filtered, and the residue was washed with acetone until no more colored substances remained. This residue was dried with suction, to give 140 g of a dried product. To this dried product was added 2.8 liters of a 0.4 M saline, and the mixture was treated at 100°C for 1 hour. Thereafter, the mixture was filtered, and the resulting residue was sufficiently washed with a 0.4 M saline, to give 3.7 liters of an extract. To this extract was added 5% cetyl pyridinium chloride until no more precipitates were formed, and the formed precipitates were harvested by centrifugation. The precipitates were suspended in a 0.4 M saline, and then centrifuged again. One liter of a 4 M saline was added to the resulting precipitates, and the mixture was treated with a homogenizer. Thereafter, 4 liters of ethanol was added thereto with stirring, and the resulting mixture was stirred for 1 hour, and thereafter filtered, to give precipitates. The steps of suspending the precipitates in 80% ethanol and thereafter filtering the suspension were repeated until the absorbance at 260 nm of the supernatant became 0. The precipitates obtained were suspended in 2 liters of a 2M saline, and insoluble matters were removed by centrifugation. The supernatant was ultrafiltered with an ultrafilter equipped with a membrane having an excluding molecular weight of 30000, and completely desalted. Thereafter, the resulting product was lyophilized, to give 3.7 g of fucoidan derived from sea cucumbers.

Reference Example 8

Six-hundred and twenty five grams of a commercially available, salt-preserved *Cladosiphon okamuranus* was suspended in 4375 ml of a 30 mM sodium phosphate buffer (pH 6.0), and treated with a homogenizer at 8000 rpm for 5 minutes. Thereafter, the homogenate was treated at 95°C for 1 hour, and centrifuged, to give supernatant. Ten grams of activated carbon was added to the resulting supernatant, and the resulting mixture was then stirred for 30 minutes, and centrifuged, to give supernatant. The resulting supernatant was concentrated to a volume of 2 liters with an ultrafilter equipped with holofiber having an excluding molecular weight of 100000, and thereafter the solvent was substituted with 20 mM sodium chloride. The resulting solution was lyophilized, to give 10.9 g of a dried product of fucoidan fraction derived from *Cladosiphon okamuranus*.

Example 1

RAW 264.7 cells (ATCC TIB 71) were suspended in a Dulbecco's modified Eagle's medium (manufactured by Bio Whittaker; 12-917 F) without phenol red containing 10% fetal bovine serum (manufactured by Gibco), 2 mM L-glutamine (manufactured by Life Technologies Oriental, 25030-149), so as to have a concentration of $6 \times 10^5$ cells/ml. The cell suspension was added to each well of a 48-well microtiter plate in a volume of 500 μl each. The cells were cultured at 37°C in the presence of 5% $CO_2$ gas for 12 hours or 24 hours. Each of aqueous solutions of the samples described below was added to each well, and the cells were cultured for a given period of time described above. Thereafter, the concentration of $NO_2^-$ formed by the oxidation of nitrogen monoxide (NO) in the medium was determined. Fraction I, Fraction II, and Fraction III of the fucoidan derived from *Kjellmaniella crassifolia* described in item (2) of Reference Example 1 and the compound represented by the formula V described in item (3) of Reference Example 2 (hereinafter referred to as 7-12SFd-F), which were used as the samples, were added so as to have a final concentration of 1, 10 or 100 μg/ml. Also, as the control, sterile distilled water was added in the same volume as that of the sample. Incidentally, as the positive control, a lipopolysaccharide (LPS, manufactured by Sigma) was added so as to have a final concentration of 1 μg/ml.

After the above culturing, 100 μl of a 4% Griess' reagent (manufactured by Sigma, G4410) was added to 100 μl of the medium, and the resulting mixture was allowed to stand at room temperature for 15 minutes. Thereafter, the absorbance at 540 nm was determined. The $NO_2^-$ concentration in the medium was calculated from a calibration curve previously drawn using $NaNO_2$ dissolved in the above medium at a known concentration. All determinations were carried out three times.

As a result, it was clarified that all of 7-12SFd-F, and Fraction I, Fraction II, and Fraction III of the fucoidan derived from *Kjellmaniella crassifolia* accelerated the induction of NO production, and had an immunopotentiating action. The results are shown in Figures 2 to 5. Concretely, Figure 2 is a diagram showing the $NO_2^-$ concentration in the medium when culturing with adding 7-12SFd-F, Figure 3 is that with adding Fraction I, Figure 4 is that with adding Fraction II, and Figure 5 is that with adding Fraction III. Also, Figure 6 is a diagram showing the $NO_2^-$ concentration in the medium when culturing with adding LPS as the positive control. In Figures 2 to 6, the axis of abscissas is the culture conditions, and the axis of ordinates is the $NO_2^-$ concentration (μM).

It was clarified from these results that Fraction I, Fraction II, and Fraction III of the fucoidan derived from *Kjellmaniella crassifolia*, and 7-12SFd-F had an induction action for NO production and an immunopotentiating effect

through the action.

Incidentally, the other fucoidans and degradation products thereof described in each of Reference Examples also exhibited a similar induction action for NO production.

In addition, the amount of IFN-γ in the same culture medium as the culture medium used for the determination of the $NO_2^-$ concentration was determined using ELISA Kit (Genzyme). However, in this system, no induction of IFN-γ production by Fraction I, Fraction II, and Fraction III of the fucoidan derived from *Kjellmaniella crassifolia,* 7-12SFd-F, and other fucoidans and degradation products thereof described in each of Reference Examples was found.

Example 2

(1) Induction Action for IFN-γ Production from Non-Stimulated Lymphocytes

An ICR mouse (female, 7-week old, body weight of about 25 g) was purchased from Japan SLC, and used for the experiment after pre-breeding the mouse for 1 week. The spleen was enucleated from the mouse, finely powdered, and suspended in RPMI-1640 medium (Gibco) containing 10% fetal bovine serum (Hiclone), to give a suspension of single cells. Adherent cells were removed by adhering them to a plastic petri dish, and non-adherent cells were used as spleen lymphocytes. Spleen lymphocytes were suspended in RPMI-1640 medium containing 10% fetal bovine serum, the concentration of which was adjusted to $2 \times 10^6$ cells/ml, and the cell suspension was added to a 96-well microtiter plate in a volume of 180 μl/well. Each sample was dissolved in distilled water, and the concentration was adjusted to 100 mg/ml. The resulting solution was diluted with the medium to a concentration of 10 times the indicated concentration. As the control, the medium was added in the same volume as that of sample. Each of fucoidan, Fraction I, Fraction II, and Fraction III of the fucoidan derived from *Kjellmaniella crassifolia* and 7-12SFd-F described in Reference Examples, which had a concentration of 10 to 500 100 μg/ml, respectively, a 100 μg/ml solution of concanavalin A (Con A; nakalaitesque) was added in an amount of 20 μg/ml to each well except for the control group, and the cells were cultured in a 5% $CO_2$ gas incubator at 37°C for 2 days or 4 days. After culturing, the culture supernatant was recovered, and the amount of IFN-γ was determined using ELISA Kit (Genzyme).

As a result, no induction action for IFN-γ production from non-stimulated lymphocytes was found for each of fucoidan, Fraction I, Fraction II and Fraction III of the fucoidan derived from *Kjellmaniella crassifolia*, and 7-12SFd-F described in Reference Examples at a dose of 500 μg/ml or less. On the other hand, a strong induction for IFN-γ production was found for the Con A-added cells.

(2) Induction Action for IFN-γ Production Under Alloantigen Stimulation

A BALB/c mouse (female, 6-week old, body weight of about 20 g) and a C57BL/6 mouse (female, 6-week old, body weight of about 20 g) were purchased from Japan SLC, and used for the experiment after pre-breeding the mouse for 1 week. The spleens were enucleated from mice having different H-2 haplotypes (BALB/c: H-2d, C57BL/6: H-2b), and spleen lymphocytes were obtained by the method described above. The cell concentration of each cell suspension was adjusted to a concentration of $2 \times 10^6$ cells/ml, and added to a 96-well microtiter plate in a volume of 100 μl each. Each of fucoidan, Fraction I, Fraction II and Fraction III of the fucoidan derived from *Kjellmaniella crassifolia*, and 7-12SFd-F described in Reference Examples which had a concentration of 10 to 500 μg/ml, respectively, and 10 μg/ml Con A was added in the same manner as in item (1) of Example 2 to each well except for the control group. As the control, the medium was added in the same volume as that of the sample. The cells were cultured in a 5% $CO_2$ gas incubator at 37°C for 4 days. After culturing, the culture supernatant was recovered, and the amount of IFN-γ was determined using the ELISA Kit.

As a result, no induction action for IFN-γ production on the lymphocytes in the state of alloantigen stimulation was found for each of fucoidan, Fraction I, Fraction II and Fraction III of the fucoidan derived from *Kjellmaniella crassifolia*, and 7-12SFd-F described in Reference Examples at a dose of 500 μg/ml or less. On the other hand, a strong induction of IFN-γ was found for the Con A-added cells.

(3) Induction Action for IFN-γ Production on Sensitized Lymphocytes Under Antigen Stimulation

A C57BL/6 mouse (female, 6-week old, body weight of about 20 g) was purchased from Japan SLC, and used for the experiment after pre-breeding the mouse for 1 week. The mouse was immunized intraperitoneally by inoculating $1 \times 10^6$ cells of Meth-A murine sarcoma cells. Fourteen days after the inoculation of the tumor, the spleen was enucleated from the mouse, and spleen lymphocytes were obtained by the method described above. The cell concentration of the cell suspension was adjusted to a concentration of $2 \times 10^6$ cells/ml, and added to a 96-well microtiter plate in a volume of 100 μl each. For the preparation of stimulated cells, mitomycin C (manufactured by KYOWA HAKKO KOGYO CO., LTD.) was added at a concentration of 50 μg/ml to Meth-A murine sarcoma cells which were suspended in RPMI-1640

medium, the concentration of which was adjusted to 2 × $10^6$ cells/ml. The mixture was treated at 37°C for 30 minutes, and washed twice. Thereafter, the cells were suspended in RPMI-1640 medium containing 10% fetal bovine serum, the concentration of which was adjusted to 2 × $10^6$ cells/ml. The prepared stimulated cells were overlaid in an amount of 100 μl/well onto each well of the plate containing spleen lymphocytes, and cultured in a 5% $CO_2$ gas incubator at 37°C for 4 days. Those used as samples were prepared and added in the same manner as that in item (1) of Example 2 so that the fucoidan derived from *Kjellmaniella crassifolia* had a concentration of 1 to 100 μg/ml; Fraction I, Fraction II and Fraction III of the fucoidan derived from *Kjellmaniella crassifolia* and 7-12SFd-F each had a concentration of 10 to 500 μg/ml; and that the positive control had a concentration of Con A 10 μg/ml. The cells were also cultured in the same manner as that in item (1) of Example 2. As the control, the medium was added in the same volume as that of the sample. After culturing, the culture supernatant was recovered, and the amount of IFN-γ was determined using the ELISA Kit. The amount of IL-12 was determined for the same culture supernatant using ELISA Kit (ENDOGEN).

The results are shown in Figures 7 and 8. Concretely, Figure 7 is a diagram showing the induction action for IFN-γ production by the fucoidan and the degradation product thereof. In the figure, the axis of ordinates is the amount of IFN-γ production (pg/ml), and the axis of abscissas is each sample and added amount (μg/ml).

Also, Figure 8 is a diagram showing the induction action for IL-12 production by the fucoidan and the degradation product thereof. In the figure, the axis of ordinates is the amount of IL-12 production (pg/ml), and the axis of abscissa is each sample and added amount (μg/ml)

As shown in Figures 7 and 8, there are exhibited enhancing action for IFN-γ production and IL-12 production on sensitized lymphocytes under antigen stimulation in a dose-dependent manner at a dose of 1 to 100 μg/ml for the fucoidan derived from *Kjellmaniella crassifolia,* and at a dose of 10 to 500 μg/ml for Fraction I, Fraction II, Fraction III and 7-12SFd-F. In addition, other fucoidans and degradation products thereof described in each of Reference Examples also exhibited similar action.

Example 3

A C57BL/6 mouse (female, 7-week old, body weight of about 20 g) was purchased from Japan SLC, and used for the experiment after pre-breeding the mouse for 1 week. The spleen was enucleated from the mouse, finely powdered, and suspended in RPMI-1640 medium (Bio Whittaker) containing 10% fetal bovine serum (Hiclone), to give a suspension of single cells. Spleen lymphocytes were suspended in RPMI-1640 medium containing 10% fetal bovine serum, the concentration of which was adjusted to 3 × $10^6$ cells/ml, and added in a volume of 10 ml to a T25 flask (Iwaki). Two identical flasks were furnished: One flask to which the medium alone was added, and the other flask to which the fucoidan derived from *Kjellmaniella crassifoliato* was added so as to have a concentration of 10 μg/ml. The cells were cultured at 37°C in the presence of 5% $CO_2$.

Ten days after the initiation of the culturing, the cells were harvested, diluted with RPMI-1640 medium containing 10% fetal bovine serum so as to have a given concentration, and added to each well of a 96-well round-bottomed microplate in a volume of 100 μl each.

As the cytotoxic activity of the cells, the amount of γ-ray liberated in the culture supernatant was determined using $^{51}$Cr-labeled EL4 thymoma cells (hereinafter referred to as EL4 cells) as target cells. Concretely, 1850 kBq of Chromium-51 Radionuclide (New England Nuclear) was added to EL4 cells, and the cells were cultured at 37°C for 1 hour and washed thrice with RPMI-1640 medium by a centrifugation step. The cells were suspended in RPMI-1640 medium containing 10% fetal bovine serum, the concentration of which was adjusted to 1 × $10^5$ cells/ml. The suspension was added to each well of a 96-well round-bottomed microplate in a volume of 100 μl each, and the cells were cultured at 37°C in the presence of 5% $CO_2$ for 5 hours. One-hundred microliters of the supernatant was taken, and thereafter the amount of γ-ray liberated in the supernatant was determined with a gamma ray scintillation counter. The cytotoxic activity was calculated as follows.

$$\text{Cytotoxic Activity (\%)} = \frac{(\text{Experimental Value} - \text{Control Value})}{(\text{Total Radioactivity Value} - \text{Control Value})} \times 100$$

Here, the total radioactivity value is the amount of γ-ray in the case where 100 μl of 0.1% Triton-X is used in

place of the cultured splenocytes. Also, the control value is the amount of γ-ray in the case where 100 μl of the medium is used in place of the cultured splenocytes, and the experimental value is the amount of γ-ray in the case where the cultured splenocytes are used.

The results are shown in Figure 9. Concretely, Figure 9 is a diagram showing cytotoxic immunopotentiating action of murine spleen lymphocytes by the fucoidan derived from *Kjellmaniella crassifolia*. The axis of ordinates is cytotoxic activity (%), and the axis of abscissas is the ratio of the effector cells of the control and the effector cells (E) which were obtained by culturing the cells, with adding the fucoidan derived from *Kjellmaniella crassifolia* described in Reference Example 1 so as to have a concentration of 10 μg/ml, to target cells (T) (E/T ratio). The bar graph shows the average value for 5 mice per each group and the standard error.

As shown in Figure 9, the cultured murine splenocytes (effector cells) exhibited a cytotoxic activity to EL4 cells (target cells) (control). In the cells cultured with adding the fucoidan so as to have a concentration of 10 μg/ml, the cytotoxic activity to EL4 cells was potentiated. The fucoidan-added group exhibited higher cytotoxic activity in spite of a lower ratio of the effector cells to target cells, so that the cytotoxic immunity was potentiated by the fucoidan.

In addition, each of other fucoidans and degradation products thereof, Fraction I, Fraction II, Fraction III, and 7-12SFd-F described in Reference Examples also exhibited similar activity.

Example 4

(1) Induction Action for IFN-γ Production from Non-Stimulated Lymphocytes

A C57BL/6 mouse (female, 6-week old, body weight of about 20 g) was purchased from Japan SLC, and used for the experiment after pre-breeding the mouse for 1 week. G-fucoidan prepared in item (2) of Reference Example 3 was added so as to have a concentration of 10 to 500 μg/ml to each well of the plate to which spleen lymphocytes prepared by the above-mentioned method were added. The cells were cultured in a 5% $CO_2$ gas incubator at 37°C for 4 days. After culturing, the culture supernatant was recovered, and the amount of IFN-γ was determined using the ELISA Kit.

As a result, no induction action for IFN-γ production by G-fucoidan was found at a dose studied.

(2) Induction Action for IFN-γ Production on Sensitized Lymphocytes Under Antigen Stimulation

A C57BL/6 mouse (female, 6-week old, body weight of about 20 g) was purchased from Japan SLC, and used for the experiment after pre-breeding the mouse for 1 week. As the sample, G-fucoidan prepared in item (2) of Reference Example 3 was dissolved in distilled water, the concentration of which was adjusted to 100 mg/ml, and diluted to a concentration of 10 times the indicated concentration with the medium. The prepared G-fucoidan was added so as to have a final concentration of 10 to 500 μg/ml to each well of the plate to which spleen lymphocytes as prepared by the above-mentioned method and Meth-A murine sarcoma cells were added. The cells were cultured at 37°C in a 5% $CO_2$ incubator for 4 days. Incidentally, as the control, the medium was added in the same volume as that of the sample and the cells were cultured. After culturing, the culture supernatant was recovered, and the amount of IFN-γ and the amount of IL-12 were determined using the ELISA Kit.

The results are shown in Figures 10 and 11. Concretely, Figure 10 is a diagram showing the induction action for IFN-γ production by G-fucoidan, wherein the axis of ordinates is the amount of IFN-γ production (pg/ml), and the axis of abscissas is sample and added amount (μg/ml). Also, Figure 11 is a diagram showing the induction action for IL-12 production by G-fucoidan, wherein the axis of ordinates is the amount of IL-12 production (pg/ml), and the axis of abscissas is sample and added amount (μg/ml). As shown in Figures 10 and 11, it was found that G-fucoidan has a potentiating action for IFN-γ production on sensitized lymphocytes under antigen stimulation in a dose-dependent manner at a dose of 10 to 500 μg/ml. As to IL-12, a marked potentiating action for production was found at a dose of 500 μg/ml.

Example 5

Action of Anti-IL-12 Antibody and Anti-Costimulation Receptor (CD 28 and CD 40) Antibody on Induction Action for IFN-γ Production by Fucoidan Derived From *Kjellmaniella Crassifolia*

C57BL/6 murine spleen lymphocytes prepared by the above-mentioned method and Meth-A murine sarcoma cells were mixed, and added to each well of a 96-well microtiter plate. The fucoidan derived from *Kjellmaniella crassifolia* as prepared in item (1) of Reference Example 1 was added to all of the wells to have a final concentration of 100 μg/ml. Further, to the wells except for the control, anti-IL-12 antibody (R & D) was added to have a final concentration of 1 μg/ml, or anti-CD 28 antibody or anti-CD 40 antibody was added to have a final concentration of 10 μg/ml. The cells were cultured at 37°C in a 5% $CO_2$ incubator for 4 days. After culturing, the culture supernatant was recovered, and the

amount of IFN-γ and the amount of IL-12 were determined by ELISA. The results are shown in Figure 12. Concretely, Figure 12 is a diagram showing an action of various antibodies on the induction action for IFN-γ production or IL-12 production by the fucoidan. In the figure, the axis of left ordinates is the amount of IFN-γ production (pg/ml), the axis of right ordinates is the amount of IL-12 production (pg/ml), and the axis of abscissas is each antibody used.

As shown in Figure 12, the induction of IL-12 production by the fucoidan derived from *Kjellmaniella crassifolia* during the antigen-presenting reaction was completely suppressed by anti-IL-12 antibody, anti-CD 28 antibody or anti-CD 40 antibody. On the other hand, the induction of IFN-γ production from the sensitized lymphocytes was about 50%-suppressed by anti-IL-12 antibody, and completely suppressed by anti-CD 28 antibody or anti-CD 40 antibody.

Example 6

Comparison of Induction Action for IFN-γ Production of Various Fucoidans

A C57BL/6 mouse was immunized by inoculating Meth-A murine sarcoma cells, and the spleen was enucleated from the mouse 24 days after the inoculation. C57BL/6 murine spleen lymphocytes as prepared by the above-mentioned method and Meth-A murine sarcoma cells were mixed, and added to each well of a 96-well microtiter plate. As the samples, the fucoidan derived from *Kjellmaniella crassifolia* as prepared in Reference Example 1, the fucoidan derived from *Cladosiphon okamuranus* as prepared in Reference Example 8, the fucoidan derived from *Fucus vesiculosus* as prepared in Reference Example 5, and the fucoidan derived from the sporophyll of *Undaria pinnatifida* as prepared in Reference Example 4 were used. Each fucoidan solution was prepared so as to have a final concentration of 10 to 500 μg/ml and added in the same manner as in item (1) of Example 2. The cells were cultured at 37°C in a 5% $CO_2$ gas incubator for 4 days. Incidentally, as the control, the medium was added in the same volume as that of the sample, and cultured. After culturing, the culture supernatant was recovered, and the amount of IFN-γ was determined using the ELISA Kit.

The results are shown in Figure 13. Concretely, Figure 13 is a diagram showing the induction action for IFN-γ production by each fucoidan. In the figure, the axis of ordinates is the amount of IFN-γ production (pg/ml), and the axis of the abscissas is each sample and added amount (μg/ml).

As shown in Figure 13, with respect to the induction ability for IFN-γ production from sensitized lymphocytes under antigen stimulation, as compared to the fucoidan derived from *Kjellmaniella crassifolia*, the fucoidan derived from *Fucus vesiculosus* was found to have the same level, and the fucoidans derived *Cladosiphon okamuranus* and the sporophyll of *Undaria pinnatifida* were found to have a weaker level.

Example 7

Four or five 5-week old male Wistar rats (Japan SLC) per one group were sensitized by intraperitoneally administering 100 μl of 0.01% ovalbumin (Sigma) in aqueous physiological saline and 100 μl of alum (trade name: Imject Alum; manufactured by Pierce). Fourteen days later, blood was obtained from the abdominal cava.

After the centrifugation (2000 rpm, 5 minutes) of the obtained blood, plasma was separated, and the amount of antigen-specific IgE was determined by a passive cutaneous anaphylaxis (PCA) using a rat. Concretely, 2-fold to 64-fold serial dilutions of plasma was prepared using physiological saline, and each dilution was injected intradermally in an amount of 0.1 ml each to the shaved back of a 7-week old male Wistar rat. Forty-eight hours after the intradermal injection, 1 ml of a mixed solution of 0.05% ovalbumin and 0.5% Evans blue (manufactured by nakalaitesque) was injected to tail vein. Thirty minutes after the tail vein injection, the rat was decapitated and allowed to exsanguinate. Blue spots appearing on the back were observed, and the maximum dilution folds were expressed as IgE titer, wherein a spot having a diameter of 5 mm or more was defined as positive.

In the group administered with the fucoidan derived from *Kjellmaniella crassifolia* as prepared in item (1) of Reference Example 1, the fucoidan derived from *Kjellmaniella crassifolia* was added at a concentration of 0.1% or 1% to a water bottle during a period of from 7 days before the day of sensitizing with antigen to the day of obtaining blood, and the rat was allowed to take water *ad libitum.* Also, in the control group, tap water was given in the same manner. As a result, an increase in the amount of antigen-specific IgE by ovalbumin sensitization was markedly suppressed by the intake of the drinking water containing 1% fucoidan derived from *Kjellmaniella crassifolia.* The results are shown in Table 1.

Table 1

| | Animal No. | IgE Antibody Titer |
|---|---|---|
| Control | 1 | 8 |
| | 2 | 64 |
| | 3 | 16 |
| | 4 | 16 |
| | 5 | 32 |
| 0.1% Fucoidan Derived From *Kjellmaniella Crassifolia* | 1 | 8 |
| | 2 | 32 |
| | 3 | 32 |
| | 4 | 32 |
| 1% Fucoidan Derived From *Kjellmaniella Crassifolia* | 1 | < 2 |
| | 2 | < 2 |
| | 3 | < 2 |
| | 4 | 4 |
| | 5 | 8 |

Example 8

Booster was given to the rats sensitized in the same manner as in Example 7 under the same conditions 19 days after priming sensitization. Blood was obtained from the abdominal cava 14 days after the final immunization. The amount of antigen-specific IgE was determined for the obtained blood by a PCA response in the same manner as above.

In the group administered with the fucoidan derived from *Kjellmaniella crassifolia* as prepared in item (1) of Reference Example 1, the fucoidan derived from *Kjellmaniella crassifolia* was added at a concentration of 0.1% or 1% to a water bottle during a period of from the day of booster to the day of obtaining blood, and the rat was allowed to take water *ad libitum*. Also, in the control group, tap water was given in the same manner. As a result, an increase in the amount of antigen-specific IgE by ovalbumin was markedly suppressed by the intake of the drinking water containing 1% fucoidan derived from *Kjellmaniella crassifolia*. It is shown from above that fucoidan is effective in therapeutic administration from the time of sensitization with an antigen as well as prophylactic administration. The results are shown in Table 2.

Table 2

| | Animal No. | IgE Antibody Titer |
|---|---|---|
| Control | 1 | 8 |
| | 2 | 32 |
| | 3 | 32 |
| | 4 | 32 |
| | 5 | 32 |
| 0.1% Fucoidan Derived From *Kjellmaniella Crassifolia* | 1 | 16 |
| | 2 | 16 |
| | 3 | 32 |
| | 4 | 32 |
| 1% Fucoidan Derived From *Kjellmaniella Crassifolia* | 1 | 2 |
| | 2 | 2 |
| | 3 | 2 |
| | 4 | 4 |
| | 5 | 2 |

Example 9

(1) The raw materials were formulated so that 1.5 g of a composition consists of 20 mg of the fucoidan derived from *Kjellmaniella crassifolia* as fucoidan, a total of 82 mg as polyphenols of a commercially available apple extract (trade name: Applephenon, manufactured by The Nikka Whisky Distilling Co. Ltd.), a commercially available grapestone extract (trade name: Gravinol, manufactured by KIKKOMAN CORPORATION), a commercially available "ten-cha" extract (trade name: Sunten-cha S, manufactured by SUNTORY LIMITED), and the balance being beef extract (manufactured by DAINIPPON PHARMACEUTICAL CO., LTD.), beer yeast (manufactured by KIRIN BREWERY COMPANY, LIMITED), lactose, corn starch (manufactured by Kato Kagaku K.K.), reduced maltose (manufactured by Towa Chemical Industry Co., Ltd.). The composition was processed in an extrusion granulator, to give a feed additive (1 mm in diameter) of the present invention, and 1.5 g each of the feed additive was made into a wrapper.

(2) The above feed additive was added as a nutritional supplement to each of the feeds in an amount of 3 wrappers per day for a large-sized canine (body weight: about 30 kg), 2 wrappers per day for a middle-sized canine (body weight: about 10 kg), or 1 wrapper per day for a small-sized canine (body weight: 5 kg).
By the intake of the feed additive of the present invention, all of the large-sized canine, middle-sized canine and small-sized canine became more active, and their appetites were enhanced. In addition, coat of fur was improved and body odor and excretion odor were reduced due to the effect of improvement in the physical conditions. These effects were markedly seen especially in senile canines, and the feed of the present invention was very useful in the improvement in physical conditions, recovery of health and rejuvenation in senile canines.

Deposited Biological Materials

(1) Name of Depository Authority
The Ministry of International Trade and Industry, National Institute of Bioscience and Human-Technology, Agency of Industrial Science and Technology
1-3, Higashi 1 chome, Tsukuba-shi, Ibaraki-ken, Japan (Zip code 305-8566)
(2) Deposited Microorganisms

(i) *Alteromonas* sp. SN-1009
Original Date of Deposit: February 13, 1996
Date of Request for Transfer to International Deposit:

November 15, 1996

Accession Number: FERM BP-5747
(ii) *Flavobacterium* sp. SA-0082
Original Date of Deposit: March 29, 1995
Date of Request for Transfer to International Deposit:

February 15, 1996

Accession Number: FERM BP-5402

INDUSTRIAL APPLICABILITY

According to the present invention, there is provided a medicament effective for a disease requiring regulation of cytokine production, a disease requiring nitrogen monoxide production and an allergic disease, wherein the medicament comprises as an effective ingredient a fucoidan and/or a degradation product thereof exhibiting an action for regulation of cytokine production, an action for induction of nitrogen monoxide production and an anti-allergic action. The medicament has an regulatory activity for production of an interleukin, an interferon, or the like in a living body, so that the medicament is useful as a therapeutic agent or prophylactic agent for a disease requiring regulation of cytokine production such as cancers and immunological diseases.
In addition, the medicament is especially useful as an agent for regulation of IFN-γ production, an agent for regulation of IL-2 production, and an agent for induction of NO production for diseases requiring applications of these agents.
The activation and enhancement of the immune system under unnecessary states may cause diseases such as allergies and autoimmune diseases in some cases. However, the cytokine enhancement and immunopotentiation by

the preparation of the present invention are selective. Therefore, the preparation of the present invention is very useful in protection in a living body because the cytokine enhancement and immunopotentiation are induced only in the case where cellular immunity is necessary to be enhanced due to the existence of antigens to be removed in the living body in a case such as a viral infection or a cancer.

In addition, the activation of excess humoral immunization is suppressed by the preparation of the present invention, so that the preparation of the present invention is very useful for suppression of allergies.

Further, a foodstuff or feed can be produced by using the fucoidan and/or degradation product thereof having an action for regulation of cytokine production, an action for induction of nitrogen oxide production and an anti-allergic action. By an intake of the fucoidan and/or degradation product thereof on a daily basis as foodstuff, there can be accomplished amelioration of symptoms of a disease requiring regulation of cytokine production, a disease requiring nitrogen monoxide production such as arteriosclerosis, an allergic disease or the like.

Therefore, the functional foodstuff or feed comprising as an effective ingredient the fucoidan and/or degradation product thereof is a functional foodstuff or feed useful for maintaining homeostasis of a living body due to the physiological action of the effective ingredient.

In addition, there is provided an agent for regulation of cytokine production, and the agent for regulation is useful for function studies on cytokine and screening for a medicament for cytokine-associated diseases.

## Claims

1. Use of a fucoidan and/or a degradation product thereof in the manufacture of a medicament for treating or preventing allergic disease.

2. The use according to claim 1, wherein the fucoidan is derived from an algae or Echinodermata.

3. The use according to claim 1 or 2, wherein the medicament is for oral administration.

4. The use according to claim 1, wherein said medicament is in the form of a food, beverage or feed.

5. The use according to claim 1, wherein said medicament is for topical administration.

## Patentansprüche

1. Verwendung eines Fucoidans und/oder eines Abbauprodukts davon für die Herstellung eines Medikaments zum Behandeln oder Vorbeugen einer allergenen Erkrankung.

2. Verwendung gemäß Anspruch 1, wobei das Fucoidan von einer Alge oder Echinodermata stammt.

3. Verwendung gemäß einem der Ansprüche 1 oder 2, wobei das Medikament für eine orale Verabreichung ist.

4. Verwendung gemäß Anspruch 1, wobei das Medikament in Gestalt eines Nahrungsmittels, Getränks oder Futters vorliegt.

5. Verwendung gemäß Anspruch 1, wobei das Medikament für äußerliche Anwendung ist.

## Revendications

1. Utilisation d’un fucoidan et/ou d’un produit de dégradation de celui-ci pour la fabrication d’un médicament pour traiter ou prévenir une maladie allergique.

2. Utilisation selon la revendication 1, dans laquelle le fucoidan est dérivé d’une algue ou d’échinodermes.

3. Utilisation selon la revendication 1 ou 2, dans laquelle le médicament est destiné à une administration orale.

4. Utilisation selon la revendication 1, dans laquelle ledit médicament est sous forme d’un aliment, d’une boisson ou d’un repas.

5. Utilisation selon la revendication 1, dans laquelle ledit médicament est destiné à une administration topique.

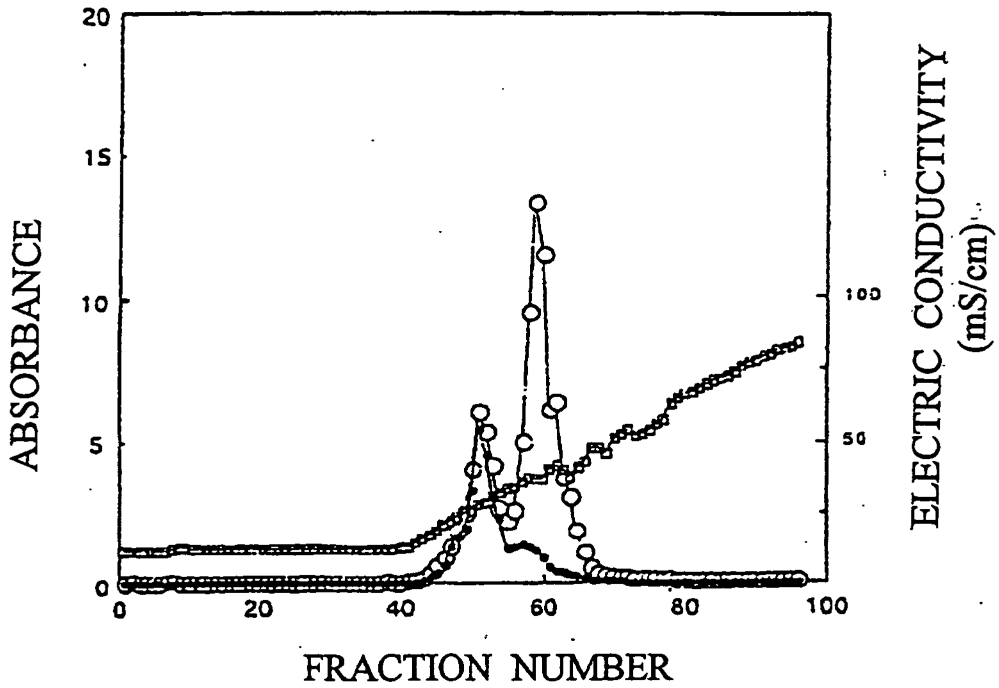
ABSORBANCE
20
15
10
5
0
0
20
40
60
80
100
FRACTION NUMBER
100
50
ELECTRIC CONDUCTIVITY
(mS/cm)

FIG. 1

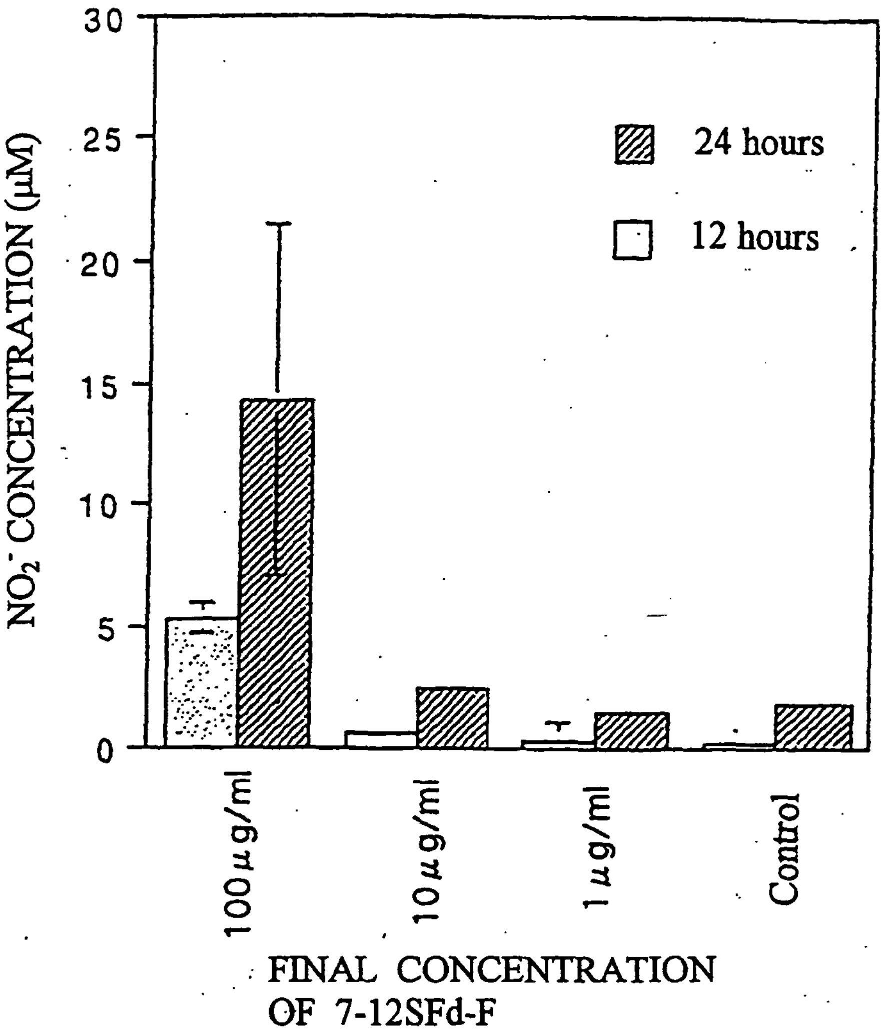
30
25
20
15
10
5
0
$NO_2^-$ CONCENTRATION (μM)
24 hours
12 hours
100μg/ml
10μg/ml
1μg/ml
Control
FINAL CONCENTRATION
OF 7-12SFd-F

FIG. 2

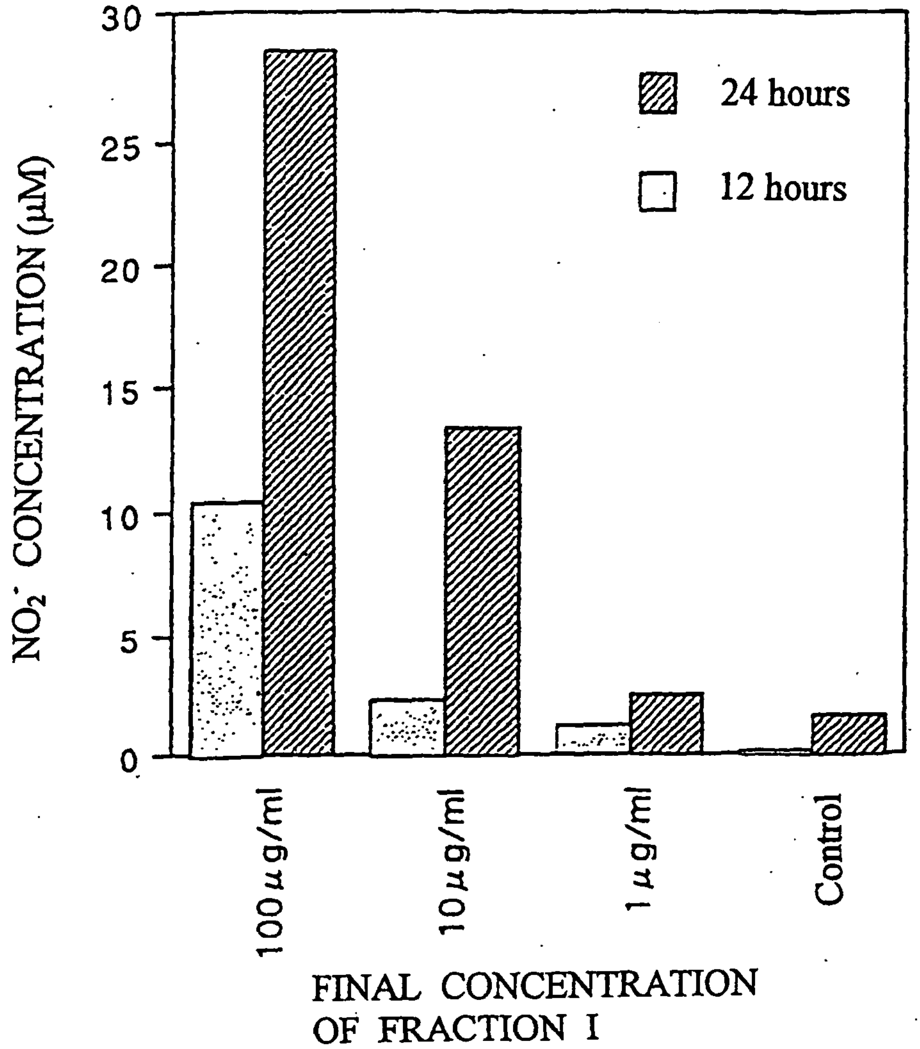
30
25
20
15
10
5
0
$NO_2^-$ CONCENTRATION (μM)
24 hours
12 hours
100 μg/ml
10 μg/ml
1 μg/ml
Control
FINAL CONCENTRATION
OF FRACTION I

FIG. 3

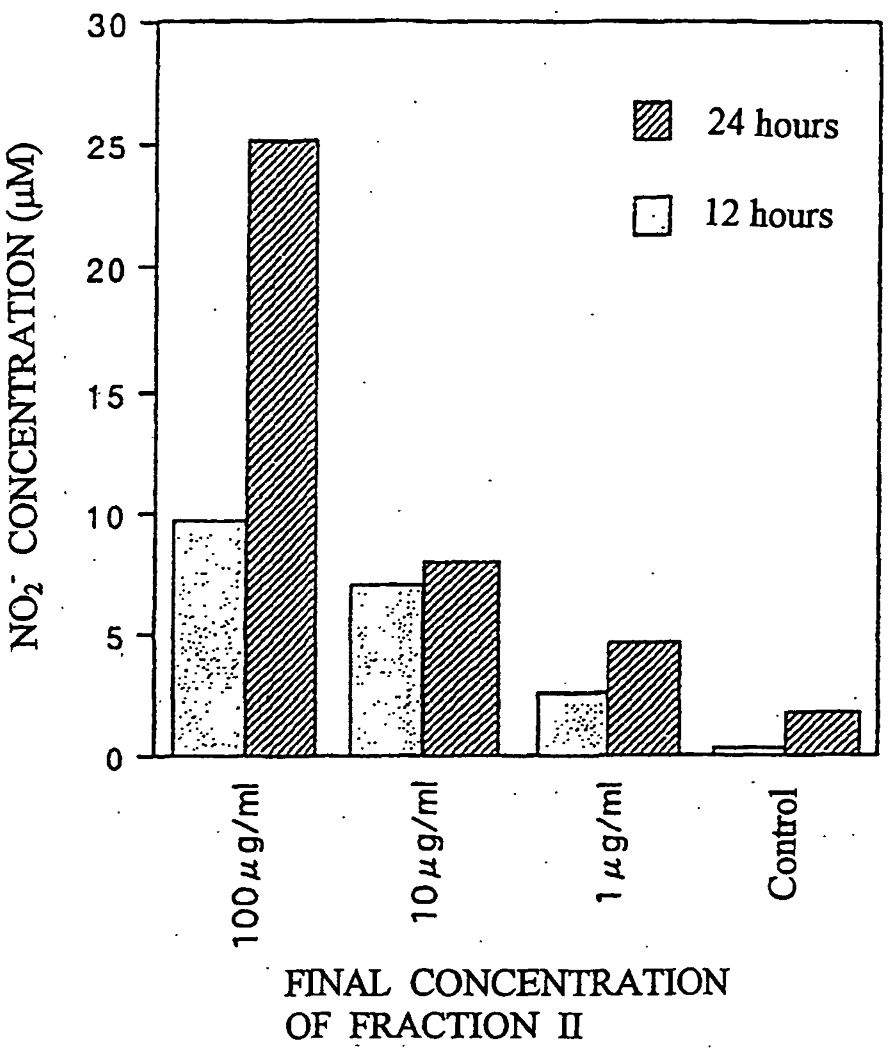
30
25
20
15
10
5
0
NO2- CONCENTRATION (µM)
24 hours
12 hours
100µg/ml
10µg/ml
1µg/ml
Control
FINAL CONCENTRATION
OF FRACTION II

FIG. 4

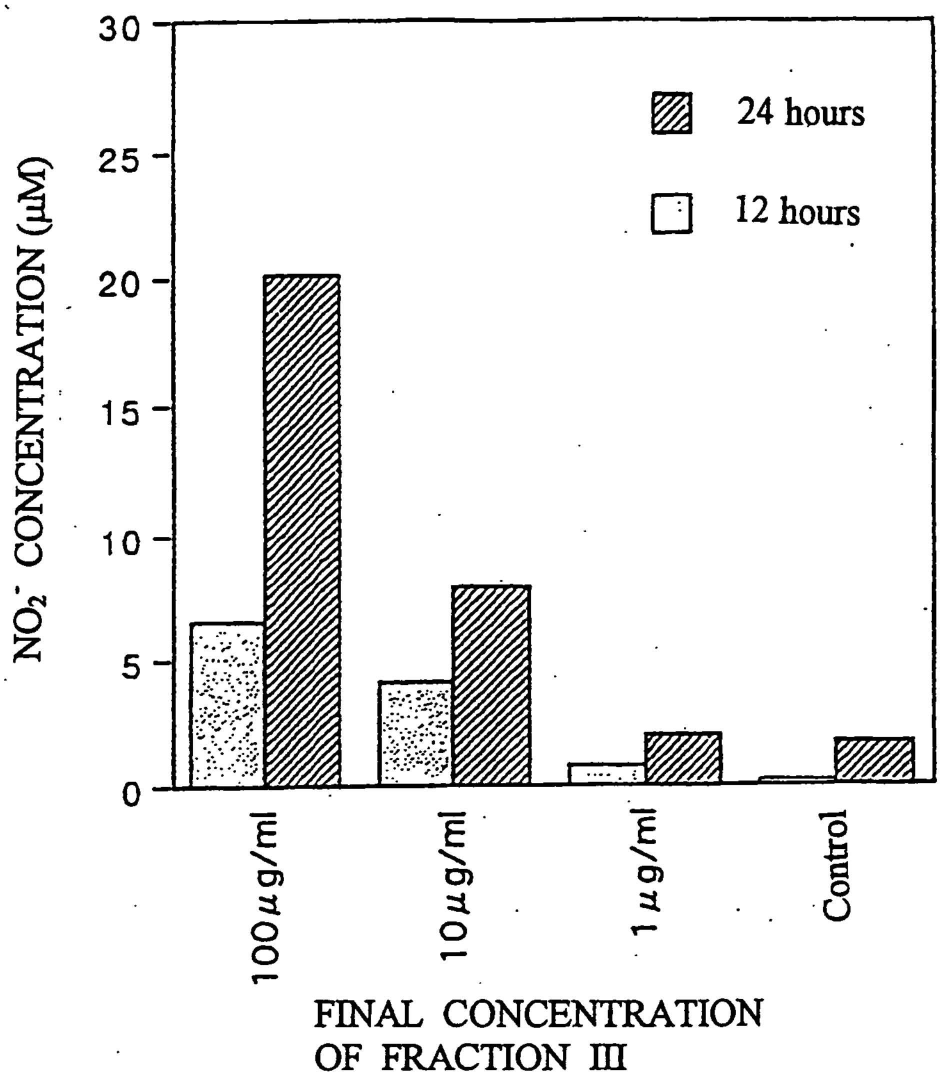
30
25
20
15
10
5
0
NO2- CONCENTRATION (μM)
24 hours
12 hours
100μg/ml
10μg/ml
1μg/ml
Control
FINAL CONCENTRATION
OF FRACTION III

FIG. 5

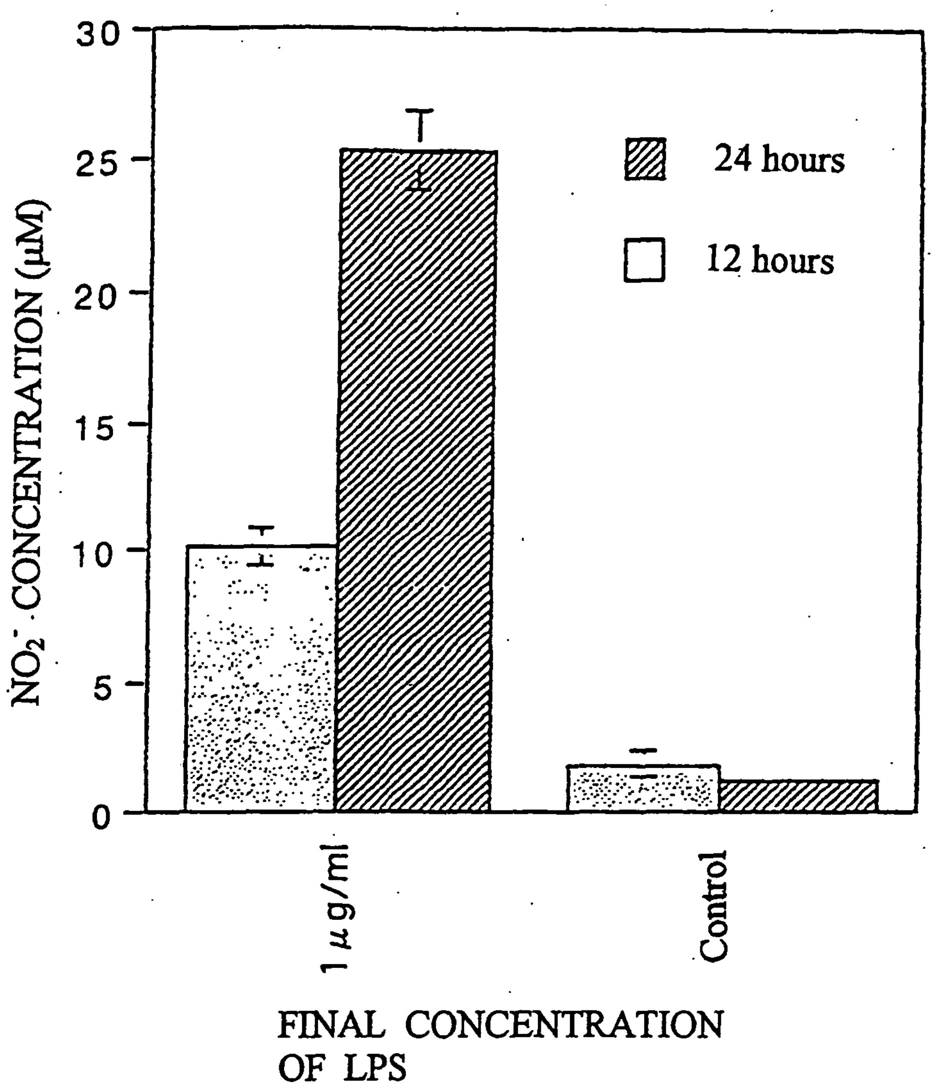
30
25
20
15
10
5
0
$NO_2^-$ CONCENTRATION (μM)
24 hours
12 hours
1 μg/ml
Control
FINAL CONCENTRATION OF LPS

FIG. 6

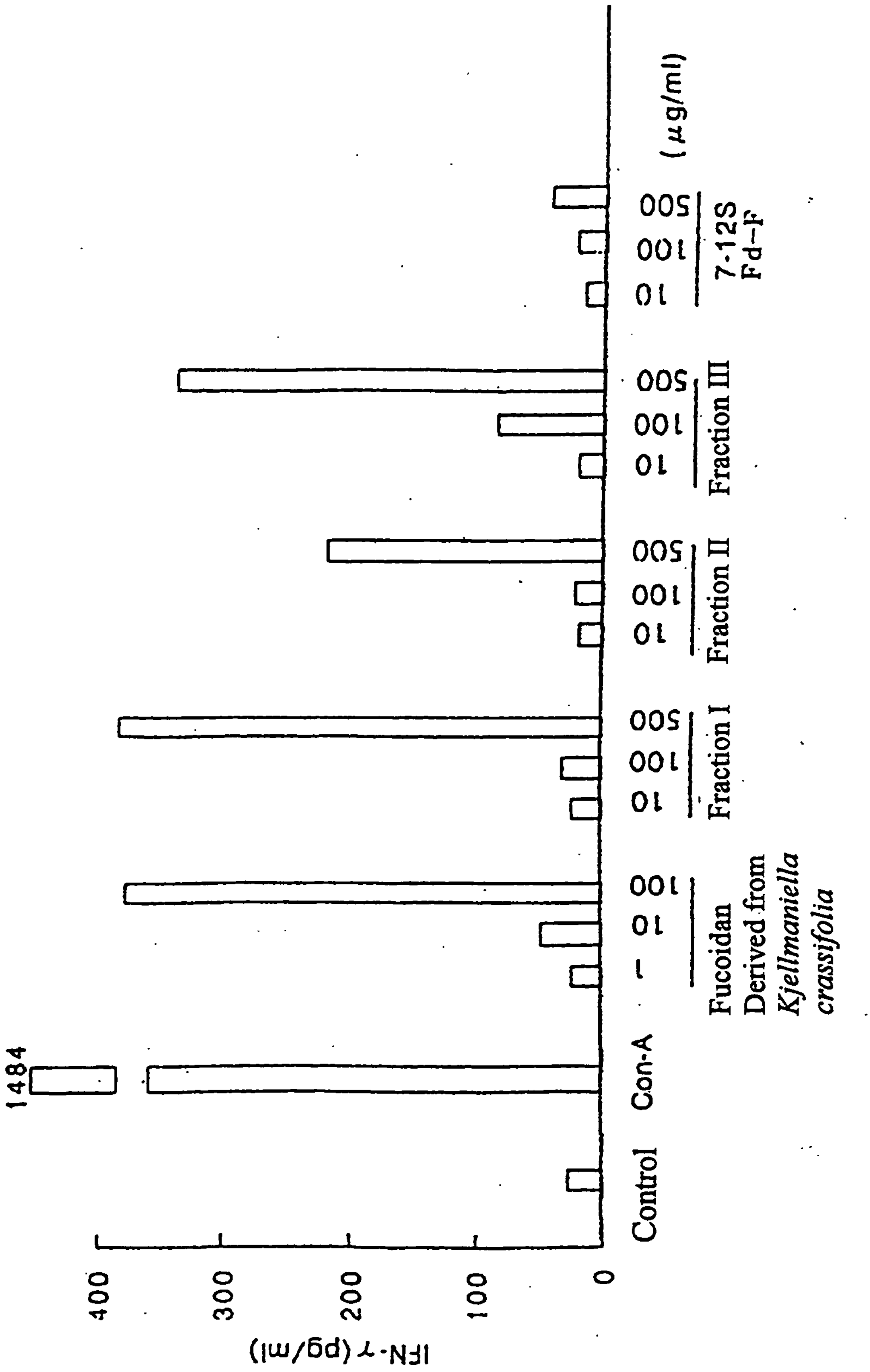
IFN-γ (pg/ml)
400
300
200
100
0
1484
Control
Con-A
1
10
100
Fucoidan
Derived from
*Kjellmaniella crassifolia*
10
100
500
Fraction I
10
100
500
Fraction II
10
100
500
Fraction III
10
100
500
7-12S
Fd-F
(μg/ml)

FIG. 7

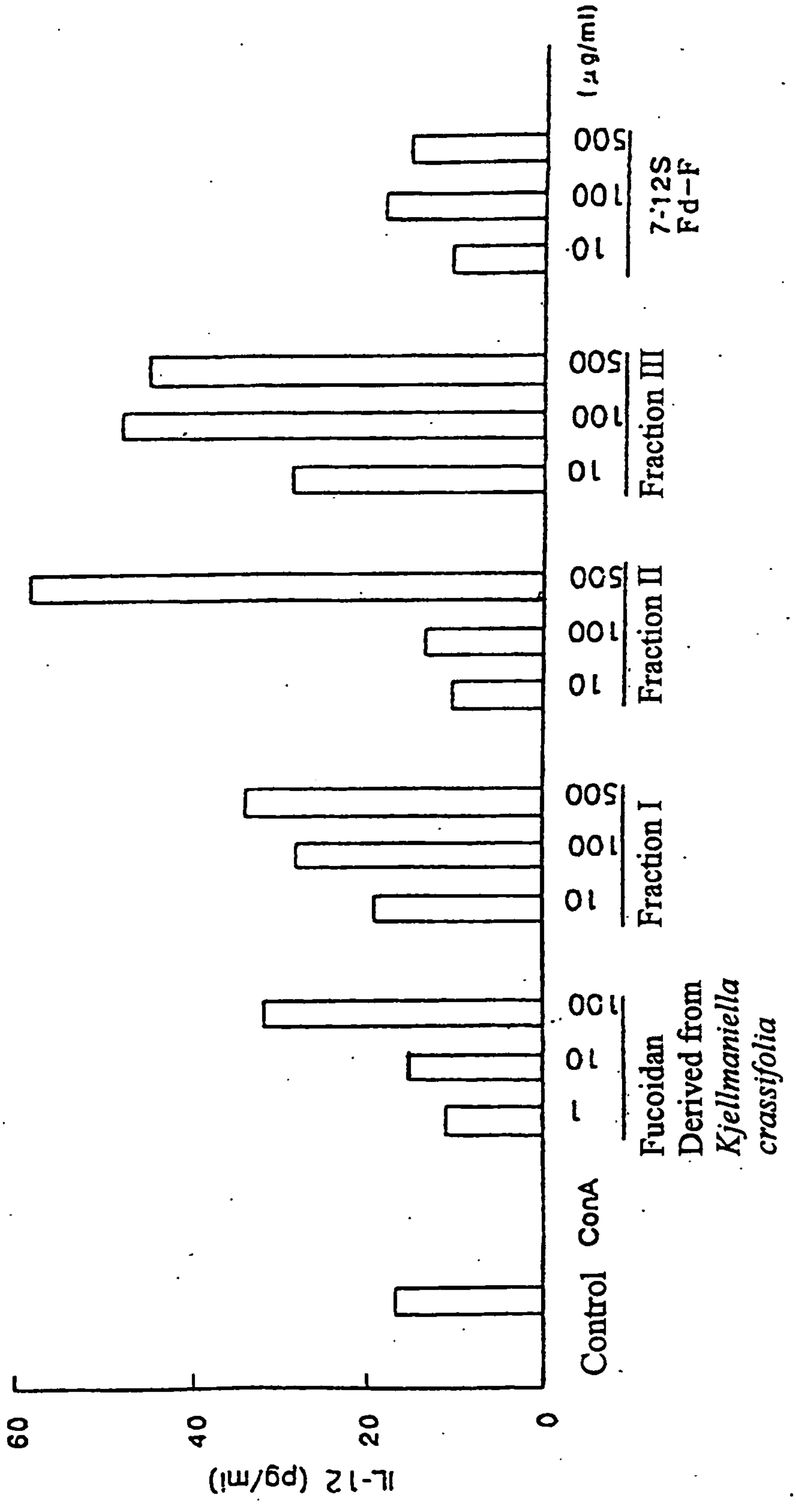
IL-12 (pg/ml)
0
20
40
60
Control
ConA
Fucoidan
Derived from
Kjellmaniella
crassifolia
1
10
100
Fraction I
10
100
500
Fraction II
10
100
500
Fraction III
10
100
500
7-12S
Fd-F
10
100
500
(μg/ml)

FIG. 8

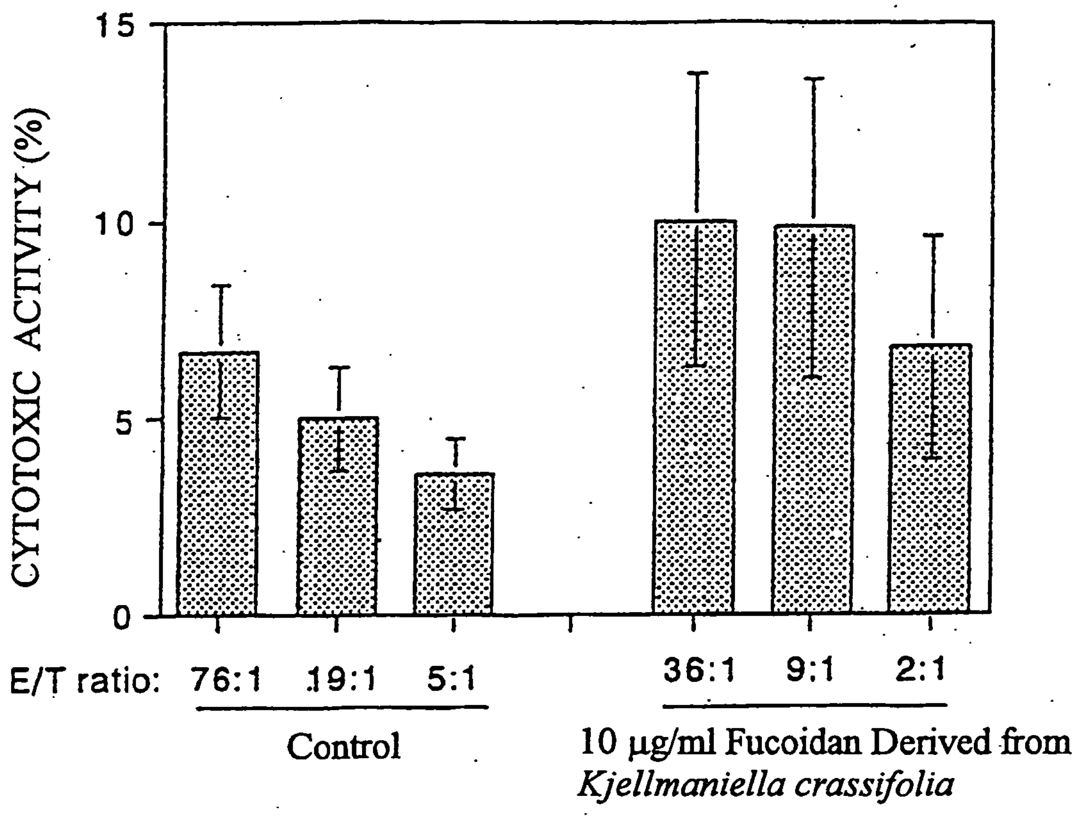
15
10
5
0
CYTOTOXIC ACTIVITY (%)
E/T ratio: 76:1 19:1 5:1
36:1 9:1 2:1
Control
10 µg/ml Fucoidan Derived from
*Kjellmaniella crassifolia*

FIG. 9

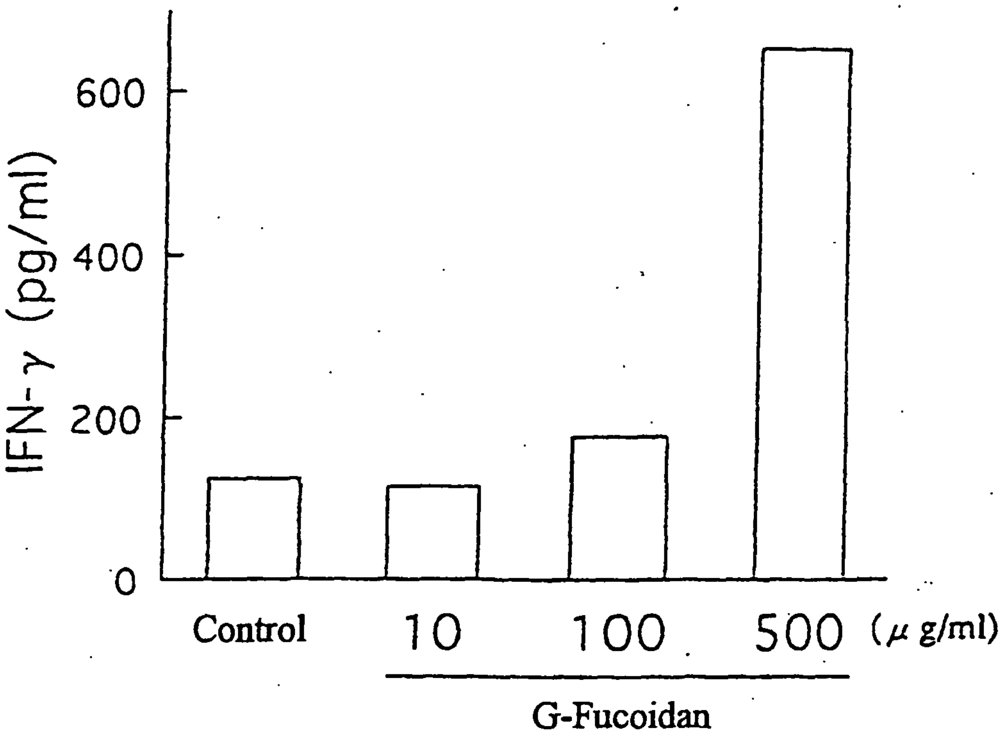


FIG. 10

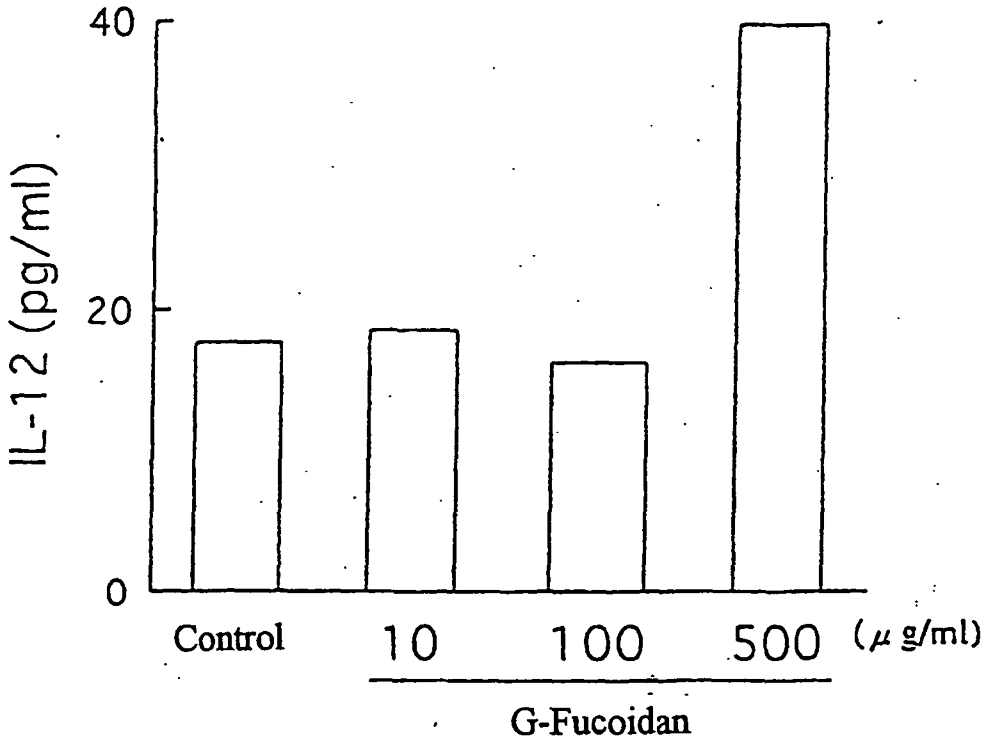


FIG. 11

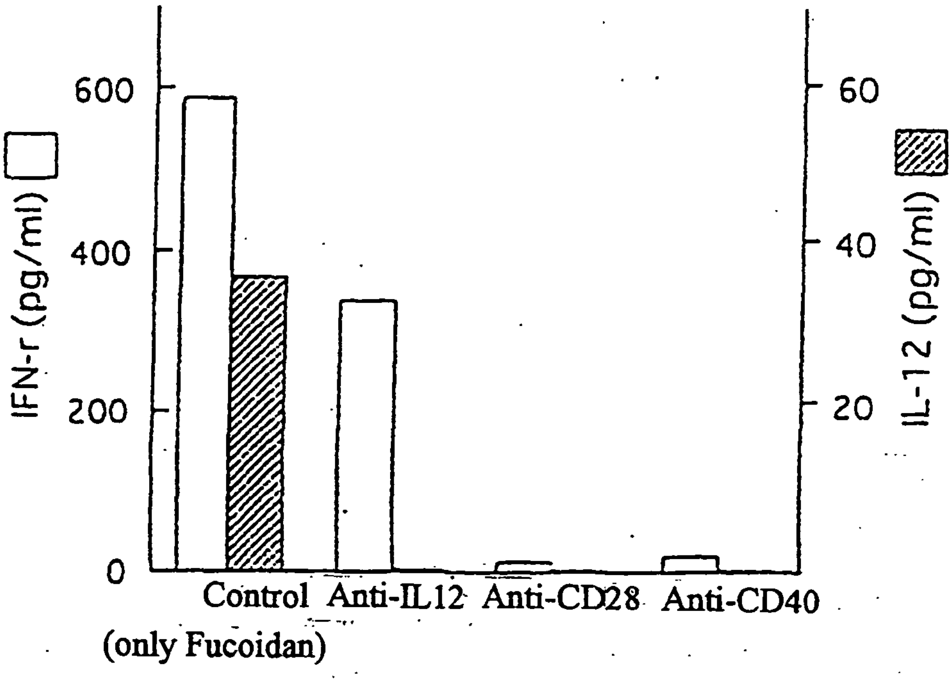
600
400
200
0
IFN-r (pg/ml)
60
40
20
IL-12 (pg/ml)
Control
(only Fucoidan)
Anti-IL12
Anti-CD28
Anti-CD40

FIG. 12

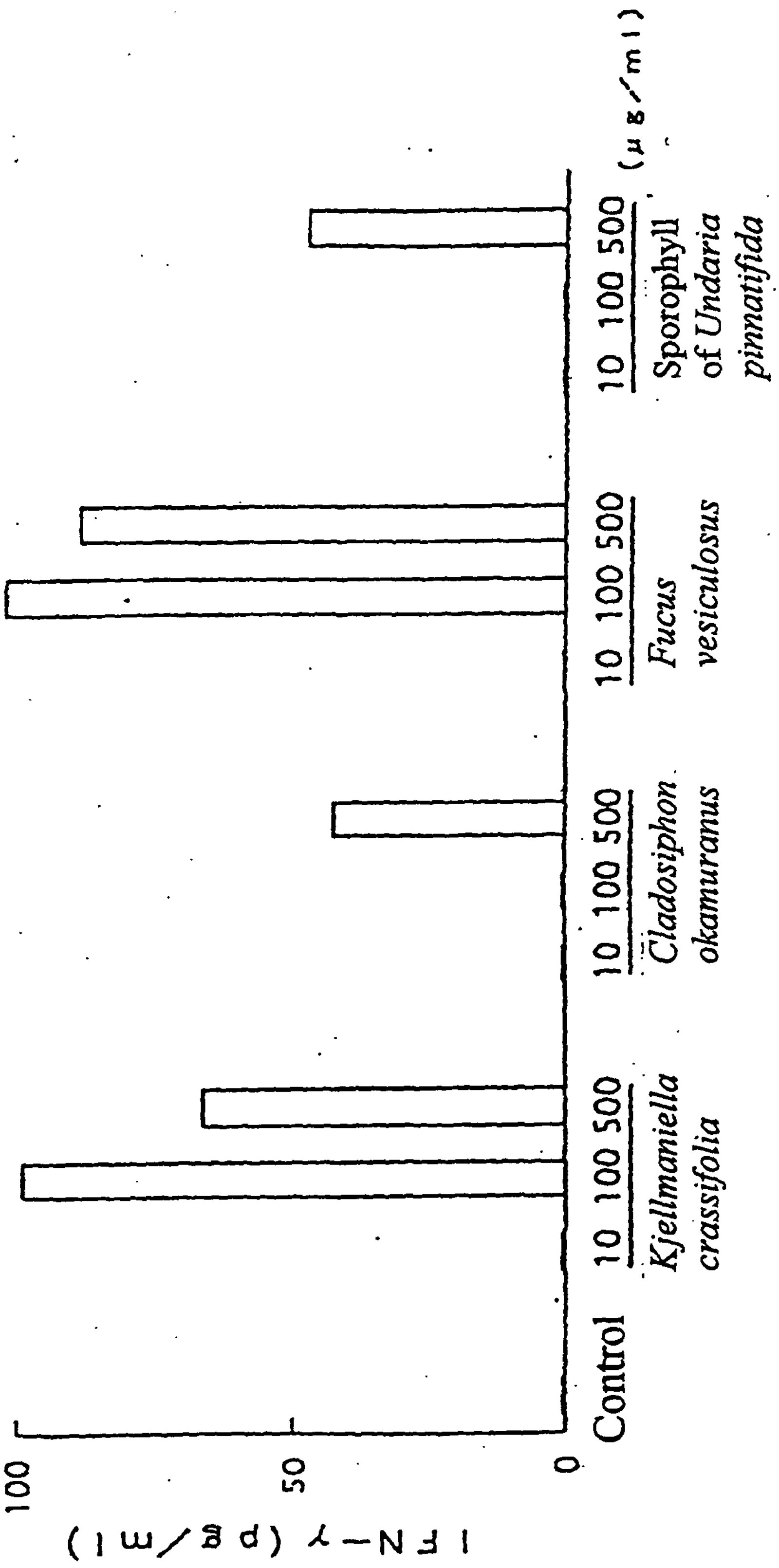
IFN-γ (pg/ml)
100
50
0
Control
10 100 500
Kjellmaniella crassifolia
10 100 500
Cladosiphon okamuranus
10 100 500
Fucus vesiculosus
10 100 500
Sporophyll of Undaria pinnatifida
(μg/ml)

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4091027 A
- WO 9726896 A
- JP 11000606 W
- JP 0000965 W


### Non-patent literature cited in the description


- *Summary of 18th Sugar Symposium,* 1996, 159
- Chugoku Kaiyo Yakubutsu Zasshi. *Zhongguo Haiyang Yaown,* 1995, 9-13
- *Analytical Biochemistry,* 1970, vol. 37, 197-202
- *Analytical Chemistry,* 1956, vol. 28, 350